# EUROPÄISCHE PATENTSCHRIFT

(11) **EP 1 150 964 B1**
(45) Veröffentlichungstag und Bekanntmachung des Hinweises auf die Patenterteilung: **29.10.2003**
(21) Anmeldenummer: 00964024.4
(22) Anmeldetag: 21.08.2000
(51) Int. Cl.: C07D 239/96, C07D 495/04, A61K 31/517, A61P 37/00

(54) **MEHRCYCLISCHE PYRIMIDIN-2,4(1H, 3H)-DIONE MIT FUNKTIONALISIERTEN ALKYLRESTEN IN 1- UND/ODER 3-POSITION, VERFAHREN ZU IHRER HERSTELLUNG UND PHARMAZEUTISCHE ZUBEREITUNGEN**
POLYCYCLIC PYRIMIDINE-2,4(1H, 3H)-DIONE WITH FUNCTIONALIZED ALKYL RADICALS IN 1- AND/OR 3-POSITION, METHOD FOR THE PRODUCTION THEREOF AND PHARMACEUTICAL PREPARATIONS
PYRIMIDIN-2,4(1H, 3H)-DIONES POLYCYLIQUES AYANT DES RESTES ALKYLE FONCTIONNALISES EN POSITION 1 ET/OU 3, LEUR PROCEDE DE PRODUCTION ET PREPARATIONS PHARMACEUTIQUES

(30) Priorität: 26.08.1999 DE 19940494
(43) Veröffentlichungstag der Anmeldung: 07.11.2001
(73) Patentinhaber: IBFB GmbH Privates Institut für biomedizinische Forschung und Beratung, 04229 Leipzig (DE)
(72) Erfinder: HERRMANN, Konrad, 04229 Leipzig (DE); LEISTNER, Siegfried, 04346 Leipzig (DE); WIPPICH, Petra, 22335 Hamburg (DE)
(74) Vertreter: Schüssler, Andrea, Dr.
(86) Internationale Anmeldenummer: EP0008126
(87) Internationale Veröffentlichungsnummer: WO01014344

(56) Entgegenhaltungen:
- EP-A- 0 454 060
- DD-A- 293 816
- DD-A- 293 824

## Beschreibung

Die Erfindung betrifft mehrcyclische Pyrimidin-2,4(1H,3H)-dione mit funktionalisierten Alkylresten in 1- und/oder 3-Position der allgemeinen Formeln la und Ib, 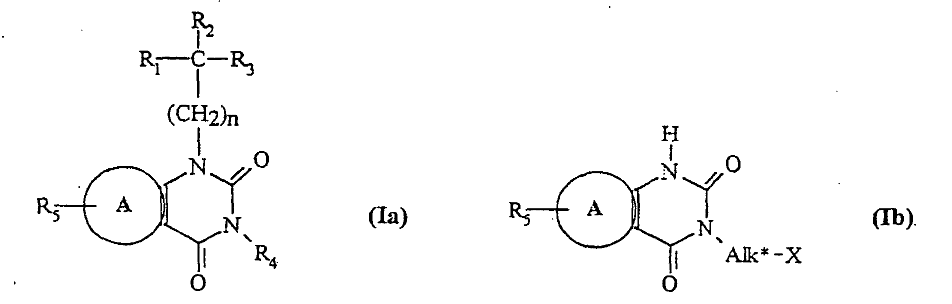 worin bedeuten:
- R¹: Wasserstoff, Methyl, Ethyl,
- R²: Wasserstoff oder Methyl,
- R³: Mercapto und -SAlkCONHOH,
- Alk: Verzweigtes oder unverzweites C₁-C₅ Alkyl(en),
- R⁴: Wasserstoff, Alkyl, Benzyl sowie Phenyl,
- n: 0, 1 oder 2,
- Alk*: Verzweigtes oder unverzweigtes C₄-C₁₂ Alkylen mit Ausnahme von 3-Methylpropylen(-CH₂-CH₂-CH(CH₃)-)),
- X: Mercapto, -SAlkCONHOH,
- A: anellierter Benzoring mit
- R⁵: Wasserstoff, 6-Methyl, 8-Methyl, 6-Fluor, 6-Chlor, 6-Brom, 6-Methylthio oder 6,7-Dimethoxy
oder
in 2,3-Position anellierter Thiophen-Ring,
der gegebenfalls in 4,5-Position mit Dimethyl substituiert oder mit einem Cyclopenten-, Cyclohexen oder Cyclohepten-Ring kondensiert ist,
sowie deren Tautomere und Salze, Verfahren zu ihrer Herstellung und pharmazeutische Zubereitungen, insbesondere zur Behandlung von Erkrankungen bei Mensch und Tier, bei denen die Inhibierung von Kollagenasen und artverwandter Metalloproteasen einen Beitrag zur Heilung bzw. Abschwächung der durch diese Enzyme verursachten Symptome bewirkt.

Verbindungen der allgemeinen Formeln la und Ib sind in der Fachliteratur bislang noch nicht beschrieben. Eine Ausnahme bildet eine Verbindung nach der allgemeinen Formel I b mit R⁵ = H und Alk* = CH₂-CH₂-CH(CH₃)- und X = SH. Diese Substanz ist als 3-(Mercapto-3-methylpropyl)chinazolin-2,4(1H,3H)dion mit Alkyl = C₄ verzweigt in der EP 0 454 060 beschrieben worden. Ihr wird eine immunstimulierende und immunrestaurative Wirkung zugeschrieben, so dass sie bei Immunerkrankungen und Virusinfektionen eingesetzt werden kann.

In der DD 293 816 beschreibt der Anmelder in Formelbild II ein Chinazolin, das für den Fall von n = 2 und R² = CH₃ als 3-( Mercapto-3-methylpropyl)-chinazolin-2,4(1H,3H)dion angesprochen werden kann. Es dient als Ausgangsstoff zur Synthese von 3-(Alkylthioalkyl)-2,4-dioxo-1,2,3,4-tetrahydrochinazolinen. Ähnlich wird in DD 293 817 verfahren, in der das Formelbild II für n = 2 und R² = CH₃ dem 3-( Mercapto-3-methylpropyl)chinazolin-2,4(1H,3H)dion entspricht und diese Substanz als Zwischenprodukt eingesetzt wird.

Es ist bekannt, daß physiologisch die enzymatische Aktivität einer strikten, abgestimmten Regulation zwischen Aktivierung und Hemmung unterliegt. Eine ungebremste enzymatische Aktivität dieser Enzyme führt besonders bei den rheumatischen Erkrankungen zum Abbau der Knorpelsubstanz und - pathophysiologisch bedeutsam - zu chronisch-schmerzhaften Veränderungen an den Gelenken.

Ein weiteres Beispiel der pathologischen Wirkung von Kollagenasen ist bei der Metastasierung von Tumoren gegeben. Sekretierte Kollagenasen bahnen sich einen Weg durch dichtes kollagenes Bindegewebe und ermöglichen es dadurch den Krebszellen aus dem Tumorverband auszuwandern und an anderer Stelle Tochtergeschwülste zu bilden.

Als weiteres relevantes medizinisch/kosmetisches Ergebnis unzureichend gebremster Einwirkung von Koliagenasen ist das UV-induzierte Erythem zu nennen, das u.a. als Folge einer intensiven Sonnenbestrahlung auftritt. Durch die UV-Strahlen des Sonnenlichtes bzw. von Bräunungsgeräten werden u.a. Kollagenasen in der bestrahlten Haut aktiviert, die in der Folge Kollagen des Bindegewebes und der Blutkapillaren spalten und dadurch für die Symptome eines Sonnenbrandes verantwortlich sind.

Bei den exemplarisch aufgezeigten pathologischen Wirkungen der enzymatischen Aktion von Kollagenasen, können deren Folgeerscheinungen durch stabile Inhibitoren verhindert werden. Es ist naheliegend, die Vorstellung zu realisieren diese Enzyme durch spezifische Inhibitoren gezielt zu hemmen, um dadurch z.B. eine fortschreitende Knorpeldestruktion bei Erkrankungen des rheumatischen Formenkreises zu unterbrechen.

Es sind bereits Verfahren zur Gewinnung von Verbindungen mit kollagenaseinhibierender Wirkung bekannt. Diese Wirkstoffe besitzen in aller Regel eine proteinogene Struktur, die eine Verwandtschaft zu natürlichen Inhibitoren aufweisen, bei denen es sich um spezielle Proteine handelt. Diese proteinogen oder pseudoproteinogenen Substratanaloga besitzen als essentielles Strukturelement eine zinkbindende Gruppe, die das Zink-Ion im aktiven Zentrum der Metallionen-abhängigen Kollagenasen chelatisiert.

Bezüglich einer therapeutischen Anwendung weisen derartige proteinogene Wirkstoffe eine Reihe von Nachteilen wie ungenügende Resorbierbarkeit, in aller Regel kurze Halbwertszeiten sowie nur eine geringe Stabilität auf.

Es besteht daher ein Bedürfnis. Arzneimittel mit nicht-proteinogener Struktur zu entwickeln, die die Nachteile proteinogener Wirkstoffe nicht aufweisen. Insbesondere besteht ein Bedürfnis nach solchen neuen Wirkstoffen mit kollagenaseinhibierenden Eigenschaften, die ausreichend stabil und gut resorbierbar sind sowie eine Affinität zum Zink-Ion des aktiven Zentrums der Kollagenasen aufweisen.

Die Erfindung hat die Aufgabe, neue chemische Substanzen mit nicht-proteinogener Struktur aufzufinden, die vorzugsweise eine kollagenaseinhibierende Wirkung aufweisen. Es ist weiter die Aufgabe dieser Erfindung, Verfahren zur Herstellung solcher Verbindungen als auch entsprechender Arzneimittel zu entwickeln, welche diese Verbindungen enthalten.

Die Aufgabe wird anspruchsgemäß gelöst. Die abhängigen Ansprüche betreffen vorteilhafte Ausführungsformen der Erfindung.

Die erfindungsgemäßen mehrcyclischen Pyrimidin-2,4(1H,3H)dione mit funktio-nalisierten Alkylresten in 1- und/oder 3-Position besitzen die allgemeinen Formeln la und Ib, 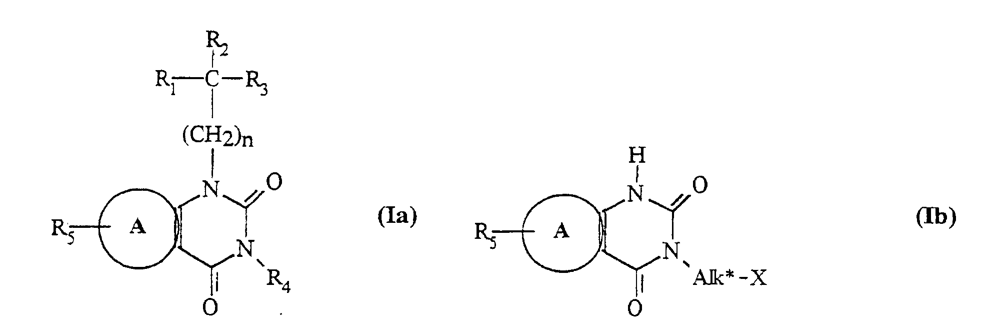 worin bedeuten:
- R¹: Wasserstoff, Methyl, Ethyl,
- R²: Wasserstoff oder Methyl,
- R³: Mercapto, oder -SAlkCONHOH,
- Alk: Verzweigtes oder unverzweigtes C₁-C₅ Alkyl(en),
- R⁴: H, Alkyl, Benzyl sowie Phenyl,
- n: 0, 1 oder 2,
- Alk*: verzweigtes oder unverzweigtes C₄-C₁₂ Alkylen mit Ausnahme von 3-Methylpropylen (-CH₂-CH₂-CH(CH₃)-) und unverzweigt),
- X: Mercapto, -SAlkCONHOH
- A: anellierter Benzoring mit
- R⁵: Wasserstoff, 6-Methyl, 8-Methyl, 6-Fluor, 6-Chlor, 6-Brom, 6-Methylthio oder 6,7-Dimethoxy
oder
in 2,3-Position anellierter Thiophen-Ring,
der gegebenfalls in 4,5-Position mit Dimethyl substituiert oder mit einem Cyclopenten-, Cyclohexen oder Cyclohepten-Ring kondensiert ist.
Unter den erfindungsgemäßen Verbindungen werden auch ihre Salze, insbesondere ihre pharmakologisch geeigneten Salze verstanden, besonders die Natrium- und Ammoniumsalze, sowie auch ihre Tautomeren.

Die Verbindungen der Formeln la und Ib sind neue chemische Substanzen mit enzyminhibitorischer, vor allem aber kollagenaseinhibierender Wirkung, aufgrund deren sie in der Human- und Veterinärmedizin vorteilhaft einsetzbar sind. Weiterhin ermöglichen diese Substanzen weiterführende Umsetzungen zu Verbindungen mit analogem oder verändertem Wirkprofil.

Von den Verbindungen der allgemeinen Formeln la und Ib sind als besondere Ausführungsform der Erfindung die Verbindungen der allgemeinen Formeln IIa und IIb hervorzuheben, 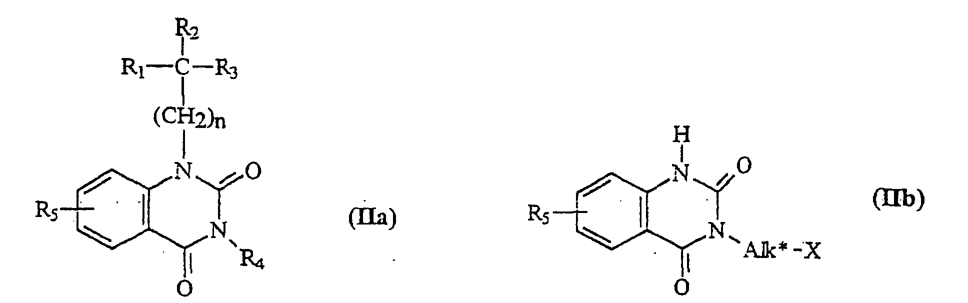 worin R¹, R², R³, R⁴, Alk, Alk*, n und X die oben genannten Bedeutungen besitzen.

Entsprechend der vorliegenden Erfindung können die Verbindungen der allgemeinen Formeln Ia und Ib sowohl als solche als auch in ihrer tautomeren Form vorliegen, ferner auch als Salze, insbesondere als pharmazeutisch unbedenkliche Alkali- oder Ammoniumsalze.

Die erfindungsgemäßen Verfahren zur Herstellung der Verbindungen der allgemeinen Formeln la, worin R₃ Mercapto und R₄ Wasserstoff bedeuten, sind gekennzeichnet durch folgende Verfahrensweisen:
(A) Umsetzung von 2-(Alkenylamino)-1-carbonsäureamiden der allgemeinen Formel III, 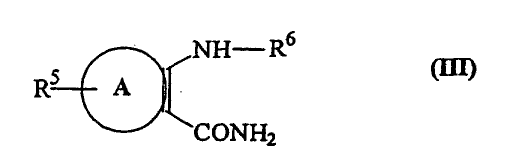 worin R₆ Alkenyl (C₃-C₆), wie z. B. Allyl, Methallyl, Crotyl, 1-Buten-4-yl, 3-Penten-1-yl und 3-Hexen-1-yl und
   A und R₅ dasselbe wie oben bedeuten,
   mit einem das -C=S-Strukturelement übertragenden Agens, wie Thiophosgen, Thiohamstoff, Ammonium- oder Alkalithiocyanat/Salzsäure, 1,1'-Thiocarbonylbisimidazol oder Benzoyl-isothiocyanat in einem polaren, aprotischen Lösungsmittel,
   - Rühren des Reaktionsansatzes,
   - Abdestillieren des Lösungsmittels im Vakuum,
   - Hinzufügen von verdünnter Alkalilauge und mäßiges Erwärmen bis etwa 60° C,
   - Abtrennen ungelöster Bestandteile durch Filtration,
   - Abkühlen und Ansäuem des Filtrates,
   - Erhitzen der erhaltenen Verbindungen der allgemeinen Formel IV, 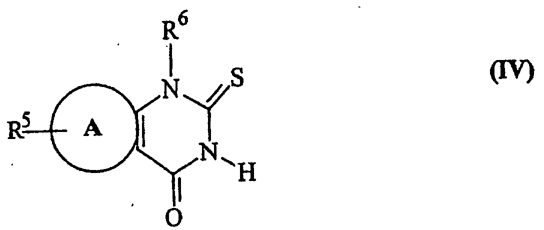 worin A, R₅ und R₆ dasselbe wie oben bedeuten,
   - mit konzentrierter Mineralsäure, wie Salzsäure, Bromwasserstoffsäure und/oder Schwefelsäure, oder Mischungen dieser Mineralsäuren mit Eisessig und/oder Ameisensäure bis zum Rückfluß,
   - Abkühlen des Reaktionsansatzes,
   - Trocknen der erhaltenen Verbindung der allgemeinen Formel V, 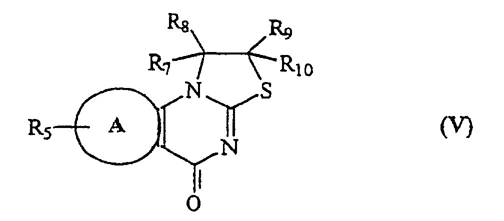 worin
      R₇, R₈, R₉ und R₁₀ Wasserstoff, Methyl oder Ethyl und A und R₅ dasselbe wie oben bedeuten,
   - bzw. Trocknen der erhaltenen Verbindung der allgemeinen Formel VI, 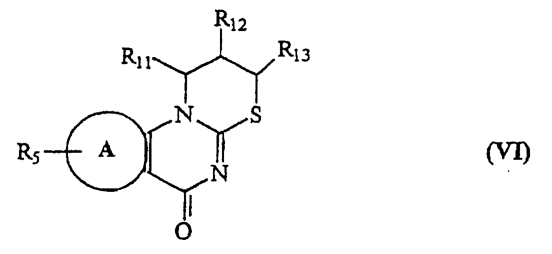 worin R₁₁, R₁₂, R₁₃ Wasserstoff, Methyl oder Ethyl und A und R₅ dasselbe wie oben bedeuten,
   - in einem Vakuum-Exsikkator über Kaliumhydroxyd oder
   - Einrühren der obigen Verbindungen in eine verdünnte, wäßrige Natriumcarbonatlösung,
   - Isolieren der Verbindungen,
   - Erhitzen dieser Verbindungen in verdünnten Mineralsäuren, wie Salzsäure, Bromwasserstorfsäure und/oder Schwefelsäure, oder Mischungen dieser Mineralsäuren mit Eisessig und/oder Ameisensäure bis zum Rückfluß,
   - Abkühlen des Reaktionsansatzes,
   - Waschen und Trocknen des Kristallisats unter Erhalt der Verbindung der allgemeinen Formel la oder ihrer Tautomeren, worin R₄ ausnahmslos Wasserstoff bedeutet.

   Das erfindungsgemäße Verfahren zur Herstellung der Verbindungen der allgemeinen Formeln la, worin
   R₃ Mercapto, R⁴ H, Alk, Benzyl sowie Phenyl,
   n 1 oder 2 und Alk, A und R₅ dasselbe wie oben bedeuten,
   ist durch folgende Verfahrensweise gekennzeichnet:
B) Umsetzung von bi- und tricyclischen 3-Alkyl(bzw.Benzyl oder Phenyl)-pyrimidin-4(3H)-on-2(1H)-thionen der allgemeinen Formel VII, 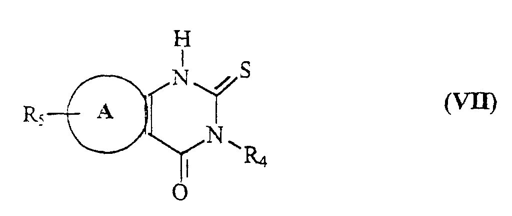 worin R⁴ H, Alk, Benzyl, Phenyl, A und R₅ dasselbe wie oben bedeuten, mit 1,ω-Dihalogenalkanen der allgemeinen Formel VIII,

   **Hal(CH**_{**2**}**)**_{**m**}**Hal** **(VIII)**

   worin
   m 2, 3 oder 4 und
   Hal Chlor, Brom oder lod bedeuten,
   in einem aprotischen, dipolaren Lösungsmittel,
   - vorzugsweise in Dimethylformamid unter Zugabe von Kaliumcarbonat. durch längeres Rühren bei Raumtemperatur,
   - Zugabe von verdünnter Salzsäure,
   - längerem Erhitzen bis zum Rückfluß,
   - Filtration der heißen Reaktionslösung,
   - Abkühlen und Aufbewahren der Lösung bei 4° C
   - unter Erhalt der Verbindungen der allgemeinen Formel la,
   worin R₃ Mercapto, R⁴ H, Alk, Benzyl, Phenyl, A und R₅ dasselbe wie oben bedeuten,
   oder
(C) Umsetzung von 2-Ammoniumcarbonsäuremethylesterthiocyanaten der allgemeinen Formel IX, 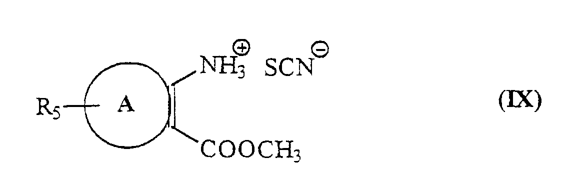 worin A und R₅ dasselbe wie oben bedeuten,
   mit 1,ω-Dihalogenalkanen der allgemeinen Formel VIII,
   worin
   m 2 oder 3 und
   Hal Chlor, Brom oder lod bedeuten,

   - Erhitzen der Reaktionspartner unter Rühren bis zum Rückfluß,
   - Abkühlen und Isolieren des Niederschlages,
   - Waschen mit Diethylether und Trocknen,
   - Lösen des Niederschlages in Wasser,
   - Filtrieren der Lösung und Zugabe von verdünnter Natronlauge bis pH 10,
   - Isolierung des ausgefallenen Niederschlages und Waschen mit Wasser,
   - Trocknen des Niederschlages und intensives Schütteln mit Chloroform,
   - Isolierung des Niederschlages und Trocknen,
   - Umkristallisieren mit einem Lösungsmittel, vorzugsweise Methylglykol, unter Erhalt der Verbindungen der allgemeinen Formel V, gewonnen durch Umsetzung mit Dibromethan,
   worin R₇, R₈, R₉ und R₁₀ Wasserstoff und A und R₅ dasselbe wie oben bedeuten,
   bzw. der Verbindung der allgemeinen Formel VI, gewonnen durch Umsetzung mit 1,3-Dibrompropan
   worin R₁₁, R₁₂, R₁₃ Wasserstoff und A und R₅ dasselbe wie oben bedeuten.
   Erhitzen der vorstehend genannten Verbindungen der allgemeinen Formel V bzw. VI in verdünnten Mineralsäuren, wie Salzsäure, Bromwasserstoffsäure und/oder Schwefelsäure, oder Mischungen dieser Mineralsäuren mit Eisessig und/oder Ameisensäure bis zum Rückfluß,
   - Abkühlen des Reaktionsansatzes,
   - Waschen und Trocknen des Kristallisats unter Erhalt der Verbindung der allgemeinen Formel la oder ihrer Tautomeren,
   worin R₁, R₂, R₄ Wasserstoff, R₃ Mercapto, n 1 oder 2 und A und R₅ dasselbe wie oben bedeuten.
   Das erfindungsgemäße Verfahren zur Herstellung der Verbindungen der allgemeinen Formel Ib,
   worin Alk* n-Butylen (-CH₂-CH₂-CH₂-CH₂-), X Mercapto und
   A und R₅ dasselbe wie oben bedeuten,
   sind durch folgende Verfahrensweise gekennzeichnet:
(D) Umsetzung von 2-lsothiocyanato-1-carbonsäureester der allgemeinen Formel X, 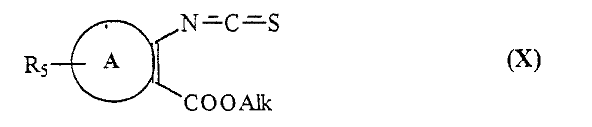 worin in Alk, A und R₅ dasselbe wie oben bedeuten,
   mit 4-Aminobutan-1-ol bei Raumtemperatur unter intensivem und längerem Rühren
   - Hinzufügen von Wasser unter Erhalt der Verbindungen der allgemeinen Formel XI, 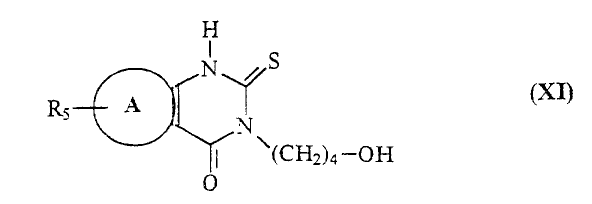 worin A und R₅ dasselbe wie oben bedeuten,
   - Umsetzung der Verbindungen der allgemeinen Formel XI
      mit konzentrierter Mineralsäure, wie Salzsäure, Bromwasserstoffsäure und/oder Schwefelsäure, oder Mischungen dieser Mineralsäuren mit Eisessig und/oder Ameisensäure bis zum Rückfluß,
   - Abkühlen des Reaktionsansatzes und Isolieren des Kristallisates,
   - Hinzufügen von wäßriger Natriumcarbonatlösung zum Kristallisat bis etwa pH 9,
   - Isolieren des Kristallisates, Waschen mit Wasser und Trocknen unter Erhalt der mehrcyclischen 2,3,4,5-Tetrahydro-7H-[1,3]thiazepino[2,3-a]pyrimidin-7-one der allgemeinen Formel XII, 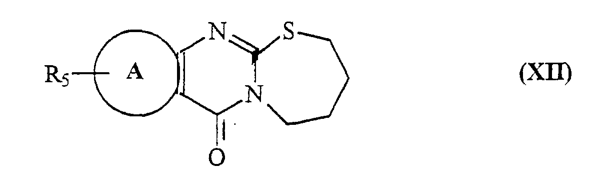
   worin A und R₅ dasselbe wie oben bedeuten.
   Erhitzen der vorstehend genannten Verbindungen der allgemeinen Formel XII in sehr verdünnten Mineralsäuren; wie Salzsäure, Bromwasserstoffsäure und/oder Schwefelsäure, oder Mischungen dieser wäßrigen Mineralsäuren mit Eisessig und/oder Ameisensäure bis zum Rückfluß,
   - Abkühlen des Reaktionsansatzes,
   - Waschen und Trocknen des Kristallisats
   unter Erhalt der Verbindung der allgemeinen Formel Ib oder ihrer Tautomeren, worin
   Alk* n-Butylen (-CH₂-CH₂-CH₂-CH₂-), X Mercapto und
   A und R₅ dasselbe wie oben bedeuten.

   Das erfindungsgemäße Verfahren zur Herstellung der Verbindungen der allgemeinen Formel Ib, worin Alk* Alkylen (C₄-C₁₂; unverzweigt und verzweigt, mit Ausnahme von 3-Methylpropylen (-CH₂-CH₂-CH(CH₃)-)), X Mercapto und A und R₅ dasselbe wie oben bedeuten,
   sind durch folgende Verfahrensweise gekennzeichnet:
(E) Umsetzung von 2-Alkoxycarbonylamino-1-carbonsäurealkylester der allgemeinen Formel XIII,

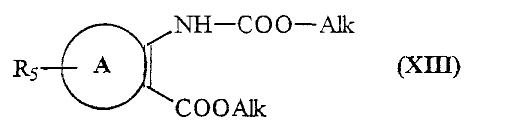

   **H**_{**2**}**NAlk*OH (XIV)**

   worin Alk Alkyl (C₁-C₃) und A und R₅ dasselbe wie oben bedeuten,
   mit Aminoalkanolen der allgemeinen Formel XIV,
   worin Alk* Alkylen (C₄-C₁₂; unverzweigt und verzweigt, mit Ausnahme von 3-Methylpropylen (-CH₂-CH₂-CH(CH₃)-)) bedeuten,
   durch Erwärmen in prinzipiell bekannter Reaktion,
   - Abkühlen des Reaktionsansatzes,
   - Zugabe von Wasser und verdünnter Salzsäure bis etwa pH 4,
   - Isolieren des Niederschlages, Waschen mit Wasser und Trocknen unter Erhalt der mehrcyclischen 3-(ω-Hydroxyalkyl)-pyrimidin-2,4(1H,3H)-dione der allgemeinen Formel XV, 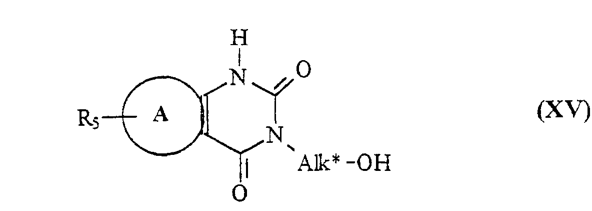 worin Alk* Verzweigtes und unverzweigtes C₄-C₁₂ Alkylen mit Ausnahme von 3-Methylpropylen (-CH₂- CH₂-CH(CH₃)-)) und A und R₅ dasselbe wie oben bedeuten.
      Umsetzung der Verbindungen der allgemeinen Formel XV mit konzentrierter Salzsäure, Phosphortrichlorid oder Phosphoroxidchlorid, vorzugsweise jedoch mit konzentrierter Bromwasserstoffsäure, durch
   - Erhitzen bis zum Rückfluß,
   - Abkühlen des Reaktionsansatzes,
   - Isolieren des Niederschlages, Waschen mit Wasser und Trocknen unter Erhalt der mehrcyclischen 3-(ω-Haloalkyl)-pyrimidin-2,4(1H,3H)-dione der allgemeinen Formel XVI, 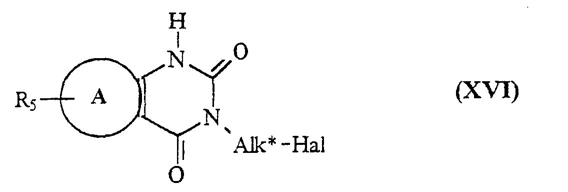
   worin Hal Chlor oder Brom und Alk*, A und R₅ dasselbe wie oben bedeuten.
   Umsetzung der Verbindungen der allgemeinen Formel XVI in einem hochsiedenden, polaren Lösungsmittel mit Thiohamstoff,
   - Erhitzen unter Rückfluß,
   - Abkühlen des Reaktionsansatzes,
   - Zugeben von Wasser und verdünnter Natronlauge bis zum Erreichen eines alkalischen pH-Wertes,
   - Nach Aufklaren der Lösung filtrieren und
   - Zugabe von verdünnter Salzsäure bis etwa pH 3,0,
   - Isolieren des Niederschlages, Waschen mit Wasser und Trocknen unter Erhalt der Verbindungen der allgemeinen Formel Ib oder ihrer Tautomeren
   worin Alk* verzweigtes und unverzweigtes C₄-C₁₂ Alkylen mit Ausnahme von 3-Methylpropylen(-CH₂-CH₂-CH(CH₃)-)), X Mercapto und A und R₅ dasselbe wie oben bedeutet.
   Das erfindungsgemäße Verfahren zur Herstellung der Verbindungen der allgemeinen Formel Ia bzw. Ib worin R₃ bzw. X jeweils SAlkCONHOH und R₁, R₂, R₄, Alk und n bzw. Alk*, A und R₅ dasselbe wie oben bedeuten, sind durch folgende Verfahrensweise gekennzeichnet:
(F) Umsetzung von Verbindungen der allgemeinen Formel la bzw. Ib,
(G)
worin R₃ und X Mercapto und R₁, R₂, R₄, Alk und n bzw. Alk* A und R₅ dasselbe wie oben bedeuten,
mit N-Hydroxyhalogencarbonsäureamiden, vorzugsweise 2-Chlor-N-Hydroxyacetamid (ClCH₂CONHOH),
- jeweils in Pyridin oder in
- acetonischer Lösung, vorzugsweise in Gegenwart einer Hilfsbase,
- bei Raumtemperatur,
- Abdestillieren des Lösungsmittels,
- Zugabe von Wasser,
- Isolieren des Niederschlages, Waschen mit Wasser und Trocknen unter Erhalt der Verbindungen der allgemeinen Formel Ia bzw. Ib oder ihrer Tautomeren, worin R₃ bzw. X SAlkCONHOH bedeutet.

Entsprechend der vorliegenden Erfindung können die unter (A-H) beschriebenen Verfahren variiert werden.

Weitere Ausführungsformen der Erfindung können beispielsweise bei der Durchführung des Verfahrens (A) darin bestehen,
- daß die Umsetzungen der Verbindungen der allgemeinen Formel III mit Benzoylisothio-cyanat in absolutiertem Aceton erfolgen.
- Bei der Durchführung des Verfahrens (B) besteht eine weitere Ausführungsformen der Erfindung darin,
- daß die Umsetzungen der Verbindungen der allgemeinen Formel VII mit den Verbindungen der allgemeinen Formel VIII in absolutiertem Dimethylformamid mit getrocknetem Kaliumcarbonat erfolgen.

Untersuchungen zur enzyminhibierenden Wirkung

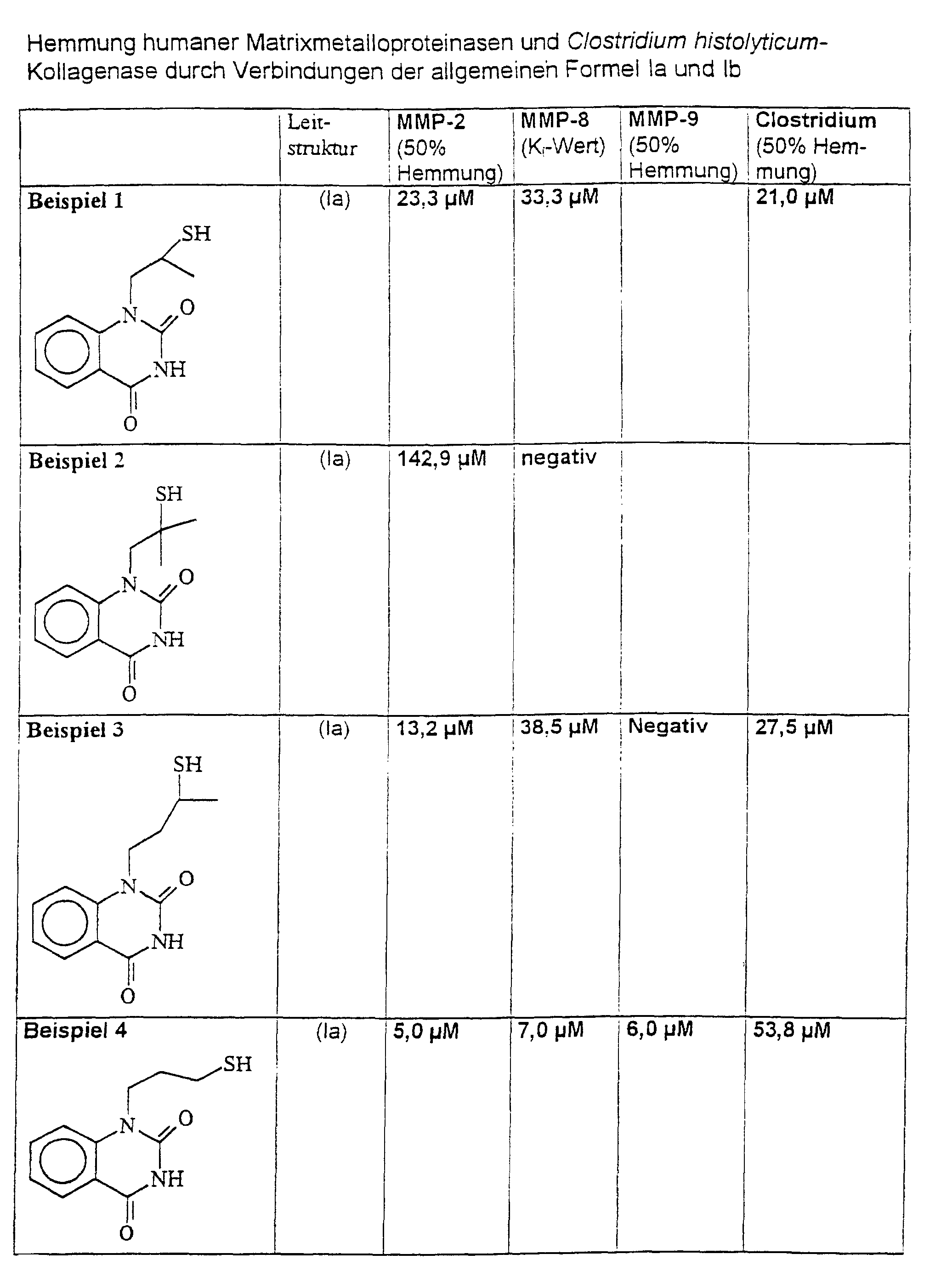

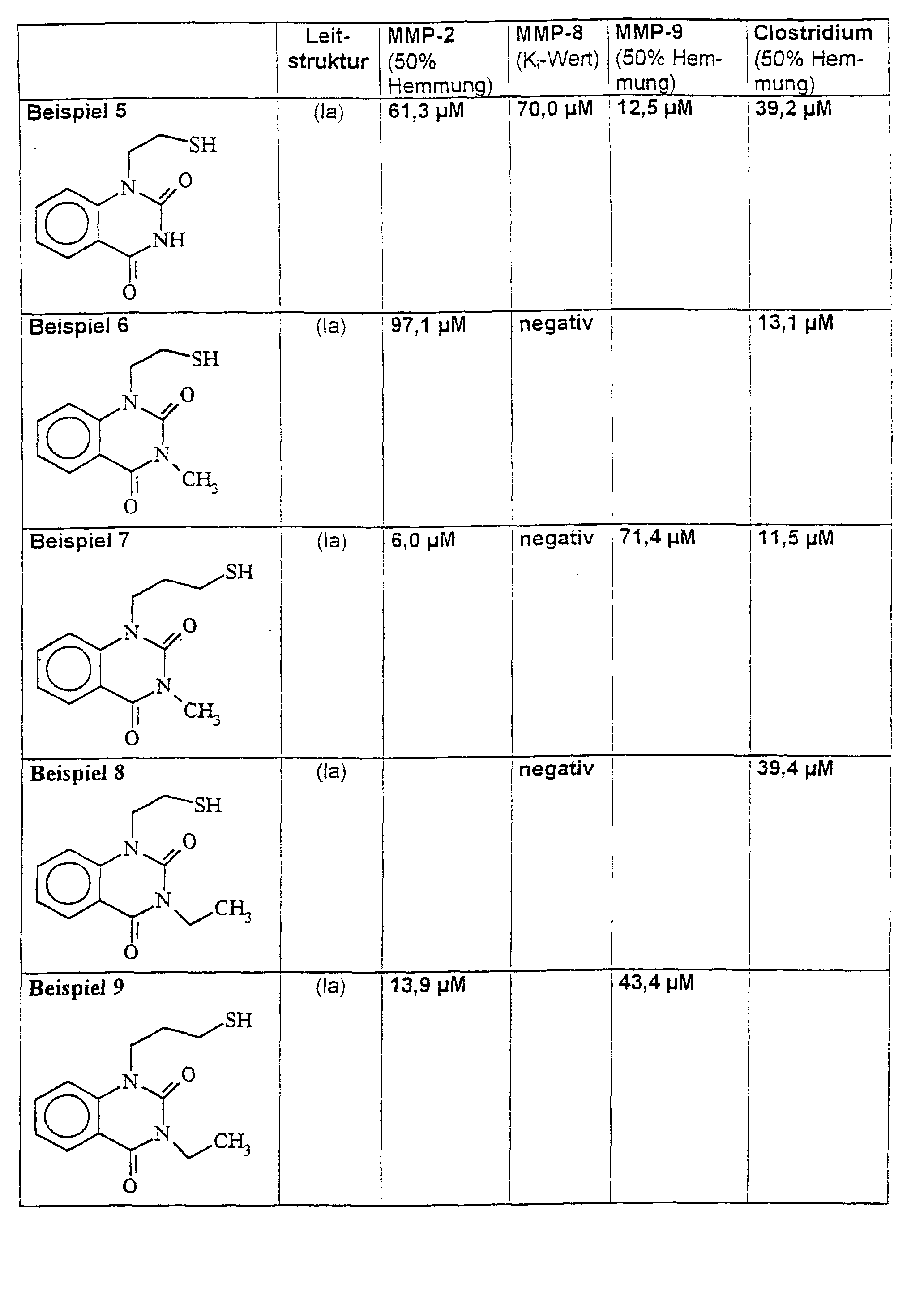

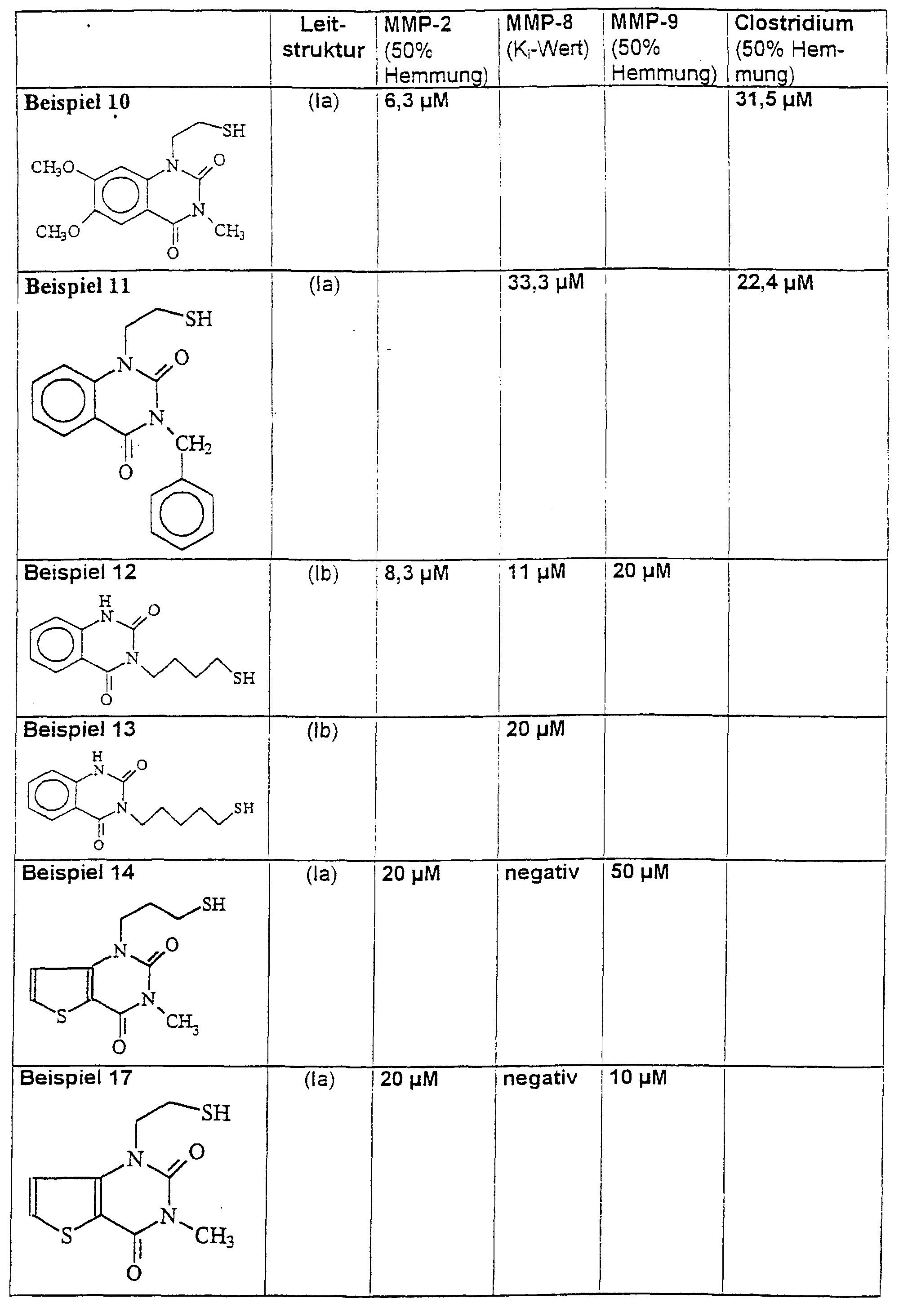

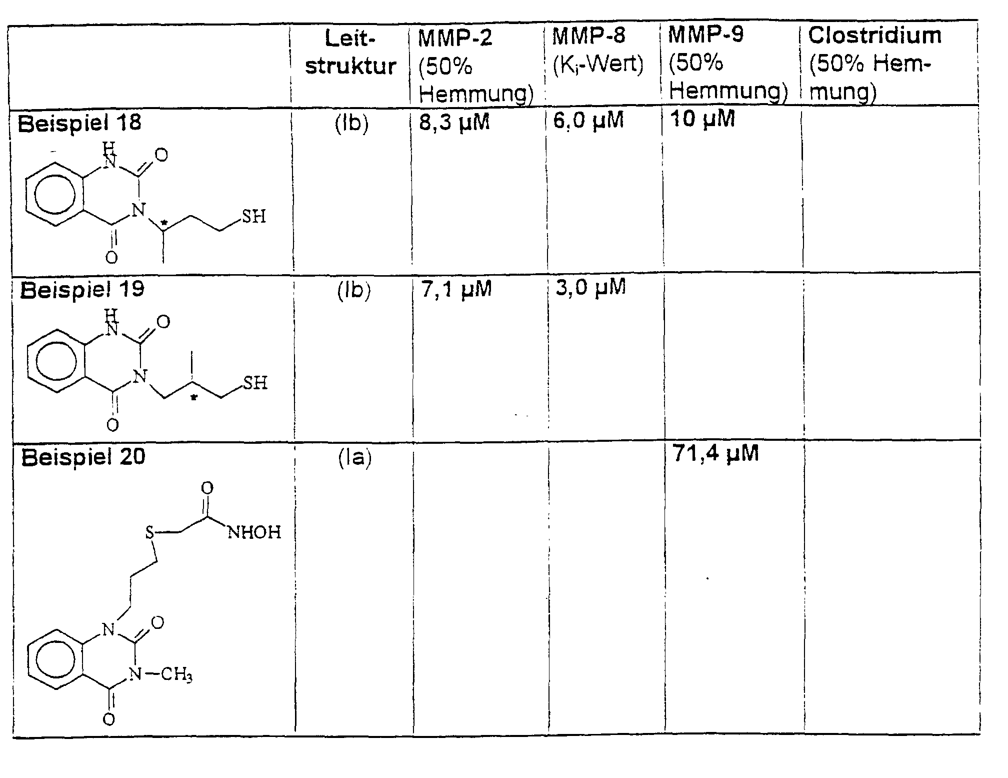

### Hemmung von Clostridium histolyticum-Kollagenase

Die Aktivität von Clostridium histolyticum-Kollagenase läßt sich in einem einfachen spektroskopischen Test mit dem Peptidsubstrat FALGPA (Furylacryoyl-Leu-Gly-Pro-Ala) bestimmen. Die Hemmung dieser Aktivität wird durch Zusatz der entsprechenden Verbindungen im Testansatz ermittelt. Enzym und Substrat sind kommerziell verfügbar.

### Hemmung von Matrix-Metalloproteinase-2 (MMP-2)

MMP-2 (Gelatinase A) wird von kultivierten dermalen Fibroblasten in beträchtlichen Mengen in das Kulturmedium abgegeben und ist daher leicht zugänglich. Die sezernierte Proform kann durch Trypsinaktivierung oder durch Behandlung mit organischen Quecksilberverbindungen in die enzymatisch aktive Form überführt werden. Für die Aktivitätsbestimmung wurde ein Screeningsystem für Kollagenaseinhibitoren auf der Basis der proteolytischen Aktivität dieses Enzyms aufgebaut.

Hierzu werden humane dermale Fibroblasten nach etablierten Standardmethoden gewonnen und kultiviert und der zellfreie Kulturüberstand mit Trypsin behandelt. Trypsin wird danach mit einem spezifischen Inhibitor (TLCK) inaktiviert und die aktive MMP-2 durch Gelfiltration an Sepharose teilgereinigt. Die Identifizierung und Charakterisierung von MMP-2 erfolgte durch die Verfügbarkeit eines kommerziellen Immuntests.

Für die Messung der MMP-2-Aktivität wurde ein nicht-radioaktives, einfaches Screeningsystem mit Gelatine als Substrat entwickelt, das die einfache Testung einer großen Zahl von Verbindungen und die kinetische Charakterisierung erlaubt. Hierzu wird die teilgereinigte MMP-2 in einer geeigneten Verdünnung mit Gelatine unter definierten und standardisierten Bedingungen (Puffer, pH, Temperatur, Inhibitorzusatz) inkubiert und nicht-gespaltene Gelatine von deren Spaltprodukten dadurch getrennt, daß diese Mischung durch ein Nitrocellulosefilter gesaugt wird. Hochmolekulare Gelatine wird an die Nitrocellulose fixiert, niedermolekulare Peptide können den Filter passieren. Die an die Nitrocellulose fixierte Gelatine läßt sich danach z.B. durch Ponceau S bzw. Coomassieblau anfärben und die Färbung quantifizieren. Für die Fixation der Reaktionsansätze kommen gebräuchliche Dot (bzw. Slot)-Blot-Apparaturen in Betracht. Die Quantifizierung der Färbung erfolgt vorzugsweise mit Hilfe eines Geldokumentationssystems erfolgen.

Die Eichung dieses Screeningsystems erfolgte durch Vergleich mit einem etablierten Kollagenase-Assay, bei dem radioaktiv bzw. fluorogen markiertes Kollagen als Substrat eingesetzt wird.

### Präparation der humanen Kollagenase MMP-9

Native MMP-9 wurde reproduzierbar und in guter Ausbeute und Reinheit aus humanem Buffy Coat gewonnen. Hierzu wird Buffy Coat mit 10% (v/v) Triton X-100 auf eine Endkonzentration von 0,4% gebracht, 30 min. auf Eis geschüttelt und dann 1 Vol. 2-fach Bindungspuffer (40 mM Tris-HCI pH 7,5, 10 mM CaCl₂, 1 M NaCl, 0,2% (v/v) Triton X-100) hinzugefügt und weitere 30 min. auf Eis geschüttelt.

Die Lösung wird 15 min. bei 16 000 Upm an einem SS-34 Rotor bei 4°C abzentrifugiert und über Glaswolle filtriert. Das Filtrat wird mit Gelatine-Agarose, welche mit Bindungspuffer äquilibriert wurde, gebatcht und 1 Std. auf Eis geschüttelt.

Die beladene Gelatine-Agarose wird in eine Säule überführt und mit mindestens 10 Vol. Bindungspuffer proteinfrei gespült. Die Elution der gebundenen MMP-9 erfolgt mit 2 Gelvolumen Bindungspuffer plus 5% (v/v) DMSO.

Das Eluat kann zum Puffer-Austausch und gleichzeitiger Abtrennung geringer Verunreinigungen an MMP-2 über eine Gelfiltration an Sephadex G-75 aufgetrennt werden. Hierzu wird der Puffer I (20 mM Tris-HCI pH 7,5, 5 mM CaCl₂, 100 mM NaCl, 0,1% (v/v) Triton X-100) verwendet.

Das so gewonnene Eluat enthällt MMP-9 in den drei bekannten Zustandsformen: Monomer, Homodimer, Heterodimer. Die Reinheit des Enzyms beträgt ca. 90%, wobei die restlichen Fremdproteine Fibronektin und äußerst geringe Mengen an TIMP's sind.

Das latente Enzym wird durch 30 - 60 min. Inkubation bei 37°C mit 1/100 Vol. Trypsin (10 mg/ml) aktiviert. Trypsin wird durch Zugabe von 1 mM PMSF oder mit einem spezifischen Inhibitor (TLCK) gehemmt.

### Klonierung und Expression der katalytischen Domäne der MMP-8

Die Nutzung der katalytischen Domäne der MMP-8 als weiteres Testenzym wurde deshalb gewählt, da es eine hohe Stabilität besitzt und darüber hinaus als aktives Enzym vorliegt und deshalb nicht aktiviert werden braucht, was als eine der häufigsten Fehlerursachen in Frage kommt, da die zur Aktivierung genutzten Quecksilberverbindungen häufig mit dem Testsystem bzw. dem Enzym interferieren und dadurch die Messergebnisse verfälschen können. Die Klonierungsstrategie richteten wir danach aus, dass wir statt des Gesamtenzyms nur dessen enzymatisch aktive katalytische Domäne in E. coli klonierten. Mit der konstruierten katalytischen Domäne der MMP-8, erzielten wir ein stabiles, enzymatisch aktives und hochreines Enzym, welches für die Routineuntersuchungen der inhibitorischen Aktivität der synthetisierten Inhibitoren sehr gut geeignet war und die Messergebnisse der einzelnen Messserien absolut vergleichbar waren.

Die Klonierung und Expression der rekombinanten katalytischen Domäne der MMP-8 wurde entsprechend den Angaben von SCHNIERER et al. (Schnierer S, Kleine T, Gote T, Hillemann A Knäuper V, Tschesche H: The recombinant catalytic domain of human neutrophil collagenase lacks type I collagen substrate specificity. Biochem Biophys Res Comm (1993) vol. 191 No. 2, 319-326) durchgeführt.

### Quantitativer Fluoreszenz-Assay für Matrix-Metalloproteinasen

Durch enzymatische Spaltung des synthetischen Substrates Mca-Pro-Leu-Gly-Leu-Dpa-Ala-Arg-NH₂ durch die jeweilige Kollagenase, wird die fluoreszente Gruppe Mca vom internen Quencher Dpa getrennt. Dabei erfolgt eine starke Zunahme der Fluoreszenz im Messansatz, die am Fluorimeter quantifiziert werden kann (λₑₓ 328 nm, λₑₘ 393 nm) und innerhalb der ersten Minuten linear verläuft. Eine gewisse Spezifität des Tests für Matrix-Metalloproteinasen ergibt sich einerseits aus der Aminosäuresequenz -Pro-Leu-Gly-Leu- im Substrat und andererseits durch die gewählten Inkubationsbedingungen. Matrix-Metalloproteinasen spalten das Substrat an der Bindung Gly-Leu. Gemessen wird die proteolytische Restaktivität vorinkubierter Ansätze von Enzym und Inhibitor, wobei die Substrat- und Enzymkonzentration konstant gehalten und die Inhibitorkonzentration variiert wurde. Es wurden für jeden getesteten Inhibitor drei Messreihen mit unterschiedlicher Substratkonzentration aufgenommen. Aus der zeitabhängigen Fluoreszenzzunahme wird die Enzymaktivität in Fluoreszenzeinheiten pro min berechnet. Die Bestimmung der Kⱼ-Werte erfolgte graphisch nach der Methode von DIXON (1953) durch Auftragung der reziproken Reaktionsgeschwindigkeit 1/v (y-Achse) gegen Inhibitorkonzentration (x-Achse).

### Assay

1984 µl Messpuffer (100mM Tris-HCI pH 7,5, 100 mM NaCl, 10 mM CaCl₂, 0,05% Brij 35)
2 µl Inhibitor, gelöst in DMSO, bzw. DMSO allein (nichtinhibierender Ansatz)
4 µl Enzym (MMP-2 oder MMP-9 oder katalytische Domäne der MMP-8)

- 5 min. Vorinkubation des Enzym-Inhibitor-Gemischs bei Raumtemperatur unter Rühren
- Start der Reaktion mit 10 µl Substrat gelöst in DMSO
- Aufzeichnung der zeitabhängigen Fluoreszenzzunahme über 2 min.

### Prüfung der Hemmwirkung durch zymographische Analyse

Durch Zymographie lassen sich komplexe Mischungen von Proteasen einfach analysieren. Dazu wird ein Proteinsubstrat (meist Casein oder Gelatine) in ein Polyacrylamidgel einpolymerisiert und die Enzymmischungen in dem entstandenen Gel elektrophoretisch aufgetrennt. Nach der Trennung werden die Gele in einem geeigneten Puffer inkubiert, wobei die Proteasen das einpolymerisierte Substrat an ihrer jeweiligen Position abbauen. Nach Fixierung und Färbung lassen sich Lysebanden identifizieren, die entsprechenden Enzymen zugeordnet werden können.

Die Wirkung der Verbindungen wurde auch in einer zymographischen Analyse überprüft. Die potentiellen Kollagenaseinhibitoren können hierbei entweder zusammen mit Gelatine in das Gel einpolymerisiert werden, oder mit der Enzymprobe nach Vorinkubation auf das Elektrophoresegel aufgetragen werden. Dies ist möglich, da die Verbindungen im Gegensatz zu proteinogenen Inhibitoren keine elektrischen Ladungen aufweisen (unterhalb des pK-Wertes der SH-Gruppen) und während der Elektrophorese nicht abgetrennt werden.

Mit einer solchen Zymographie können komplexe Quellen von Kollagenasen untersucht werden. Im besonderen ist die Analyse der Wirkung der neu synthetisierten Verbindungen auf die Kollagenasen von Zellkulturüberständen, von Synovialflüssigkeit und von Gewebeextrakten durchgeführt worden. Die Unterscheidung von metallabhängigen Proteasen und anderen in diesen komplexen Lösungen vorhandenen proteolytischen Enzymen kann durch die spezifische Hemmung mit Phenanthrolin erfolgen.

Die Erfindung wird durch die nachfolgenden Beispiele näher erläutert;

### Beispiel 1

(R,S)-1-(2-Mercaptopropyl)-chinazolin-2,4(1H,3H)-dion
(Formel la, R¹ = Wasserstoff, R² = Methyl, R³ = Mercapto, R⁴ = Wasserstoff, n = 1, A = Benzoring mit R⁵ = Wasserstoff)
a) 2-Allyiaminobenzoesäureamid (Formel III, R⁶ = Allyl, A = Benzoring mit R⁵ = Wasserstoff)
   13.60 g (0,1mol) 2-Aminobenzamid werden in 80 ml Wasser suspendiert. Der Suspension werden 6,90 g Kaliumcarbonat sowie 7,70 g (0,1mol) Allylchlorid hinzugefügt. Danach wird der Reaktionsansatz 2 Stunden am Rückflußkühler erhitzt. Nach dem Erkalten wird das ausgefallene Kristallisat aus Ethanol/Wasser (1:1; v/v) umkristallisiert. Es werden 13,8 bis 14,3 g 2-Allylaminobenzoesäureamid erhalten.
   Farblose Kristalle.
   C₁₀H₁₂N₂O (176,2). F.: 88-92°C (Ethanol/Wasser).
   Ausbeute: 79%.
b) 1-Allylchinazolin-4(3H)-on-2(1H)-thion (Formel IV, R⁶ = Allyl, A = Benzoring mit R⁵ = Wasserstoff)
   8,81 g (0,05mol) der nach a) hergestellten dc-reinen Verbindung werden in 50 ml abs. Aceton gelöst und mit 0,06mol Benzoylisothiocyanat versetzt. Der Reaktionsansatz wird 24 Stunden bei Raumtemperatur gerührt. Anschiießend wird im Vakuum das Lösungsmittel abdestilliert. Zu dem Rückstand werden zunächst 50 ml Wasser und danach 10%ige Natronlauge bis pH 9 unter Rühren hinzugefügt. Unter Rühren wird der Ansatz langsam auf 60°C erwärmt und von wenig ungelösten Anteilen durch Filtrieren befreit. Nach Abkühlen der Lösung wird mit 10%iger Salzsäure angesäuert. Der gebildete Niederschlag wird abgesaugt, mit Wasser gewaschen und nach dem Trocknen umkristallisiert. Es werden 5,35 g
   1-Allylchinazolin-4(3H)-on-1(H)-thion erhalten. Hellgelbe Kristalle.
   C₁₁H₁₀N₂OS (218,3). F.: 179-180°C (Ethanol).
   Ausbeute: 49%.
   IR (υ in cm-1): 1608 (NH), 1692 (C=O), 2948 (CH2).
   MS m/e (%B): M⁺ 218(27), 203(100), 119(21), 77(33).
c) (R,S)-2-Methyl-1,2-dihydro-5H-thiazolo[3,2-a]chinazolin-5-on-hydrobromid (Formel V, R⁷ = R⁸ = R¹⁰ Wasserstoff, R⁹ = Methyl, A = Benzoring mit R⁵ = Wasserstoff)
   2,18 g (0,01mol) der nach b) hergestellten dc-reinen Verbindung werden mit 20 ml konz. Bromwasserstoffsäure 30 Minuten am Rückflußkühler erhitzt. Der nach dem Erkalten gebildete Niederschlag wird abgefrittet und im Vakuum-Exsiccator über Kaliumhydroxid getrocknet. Es werden 1,67 g (R,S)-2-Methyl-1,2-dihydro-5Hthiazolo[3.2-a]chinazolin-5-on-hydrobromid als dc-reine Verbindung erhalten. Farblose Kristalle.
   C₁₁H₁₀N₂OS x HBr (298,3). F.: 242-247°C (Ethanol).
   IR (υ in cm-1): 1618 (C=N), 1686 (C=O), 2922 (CH₂).
   MS m/e (%B): M⁺ 218(100), 203(13), 190(49), 175(17).
d) (R,S)-1-(2-Mercaptopropyl)-chinazolin-2,4(1H,3H)-dion (Formel la,
   R¹ = Wasserstoff, R² = Methyl, R³ = Mercapto, R⁴ = Wasserstoff, n = 1,
   A = Benzoring mit R⁵ = Wasserstoff)
   1,50 g der nach c) hergestellten Verbindung werden in eine aus 0.6 ml konzentrierter Schwefelsäure, 1,5 ml Eisessig und 66 ml Wasser bereitete Säuremischung eingetragen und am Rückflußkühler 8 Stunden erhitzt. Der nach dem Erkalten gebildete Niederschlag wird abgefrittet, mit wenig Wasser gewaschen und nach dem Trocknen umkristallisiert. Es werden 1,05 g (R,S)-1-(2-Mercaptopropyl)-chinazolin-2,4(1H,3H)-dion erhalten.
   Farblose Kristalle.
   C₁₁H₁₂N₂O₂S (236,3). F.: 170-173°C (Ethanol).
   Ausbeute: 89%.
   IR (υ in cm⁻¹): 1608 (NH), 1682 (C=O), 1702 (C=O), 2554 (SH), 2922 (CH₂).
   MS mle (%B): M^{+.} 236(6), 204(21), 176(17), 162(89), 149(34), 132(100).
   UV_{Ethanol} (in nm)(log ε): 220.2 (4.83), 244,2 (4.22), 310.8 (3.94).

### Beispiel 2

1-(2-Mercapto-2-methyl-propyl)-chinazolin-2.4(1 H,3H)-dion
(Formel la, R¹ = R² = Methyl, R³ = Mercapto, R⁴ = Wasserstoff, n = 1,
A = Benzoring mit R⁵ = Wasserstoff).
Farblose Kristalle. C₁₂H₁₄N₂O₂S (250,3). F.: 183-186°C (Ethanol).
Ausbeute: 76% (bezogen auf den letzten Syntheseschritt).
IR (υ in cm-1): 1610 (NH), 1684 (C=O), 1712 (C=O), 2552 (SH), 2928 (CH₂).
MS m/e (%B): M^{+.}+1 251(100), M^{+.} 250(8), 217(9), 176(58), 162(33), 149(100), 132(50).
UV_{Ethanol} (in nm)(log ε): 220.0(4.36), 245,2 (3.80), 312.2 (3.61).

Die Darstellung dieses Mercapto-Derivates erfolgte analog Beispiel 1.
Dabei wurden folgende Zwischenstufen isoliert und charakterisiert:
a) 2-Methallylaminobenzoesäureamid (Formel III, R⁶ = Methallyl, A = Benzoring mit R⁵ = Wasserstoff).
   Dargestellt aus 2-Aminobenzamid und Methallylchlorid. Ausbeute: 87%.
   Farblose Kristalle. C₁₁H₁₄N₂O (190,2). F.: 130°C (Ethanol/Wasser).
   IR (υ in cm-1): 1622 asymm.(NH, C=O), 2910 (CH₂).
   MS m/e (%B): M⁺ 190(50), 173(86), 158(19), 144(47), 132(100).
b) 1-Methallylchinazolin-4(3H)-on-2(1H)-thion (Formel IV, R⁶ = Methallyl, A = Benzoring mit R⁵ = Wasserstoff).
   Dargestellt aus 2-Methallylaminobenzoesäureamid und Benzoylisothiocyanat in abs. acetonischer Lösung. Ausbeute: 53%.
   Hellgelbe Kristalle. C₁₂H₁₂N₂OS (232,3). F.: 181-182°C (Ethanol).
   IR (υ in cm⁻¹): 1606 (NH), 1682 (C=O), 2930 (CH₂).
   MS m/e (%B): M⁺ 232(31), 217(100), 199(27), 184(11), 119(13).
c) 2,2-Dimethyl-1,2-dihydro-5H-thiazolo[3,2-a]chinazolin-5-on (Formel V, R⁷ = R⁸ = Wasserstoff, R⁹= R¹⁰ = Methyl, A = Benzoring mit R⁵ = Wasserstoff).
   Dargestellt aus
   1-Methallylchinazolin-4(3H)-on-2(1H)-thion und konz. Bromwasserstoffsäure. Der nach dem Erkalten gebildete Niederschlag wird bei Raumtemperatur mit 5%iger Natriumcarbonatlösung digeriert. Ausbeute: 87%.
   Farblose Kristalle. C₁₂H₁₂N₂OS (232,3). F.: 269°C (Ethanol).
   IR (υ in cm⁻¹): 1618 (NH), 1684 (C=O), 2923 (CH₂).
   MS m/e (%B): M⁺ 232(100), 217(15), 204(18), 189(38), 171(26).
   UV_{Ethanol} (in nm)(log ε): 234.0 (4.31), 254.5 (4.38), 309,5 (3.90).

Die Umsetzung der nach c) gewonnenen Verbindung analog der unter 1d) aufgeführten Verfahrensweise liefert 1-(2-Mercapto-2-methyl-propyl)-chinazolin-2,4(1H,3H)-dion.

### Beispiel 3

(R,S)-1-(3-Mercaptobut-1-yl)-chinazolin-2,4(1H,3H)-dion
(Formel Ia, R¹ = R⁴ = Wasserstoff, R² = Mercapto, R³ = Methyl, n = 2,
A = Benzoring mit R⁵ = Wasserstoff).

Farblose Kristalle. C₁₂H₁₄N₂O₂S (250,3). F.: 142-143°C (Ethanol).
Ausbeute: 86% (bezogen auf den letzten Syntheseschritt).
IR (υ in cm-1): 1610 (NH), 1680 (C=O), 1694 (C=O), 2554 (SH).
MS m/e (%B): M^{+.}+1 251(100).

Die Darstellung dieses Mercapto-Derivates erfolgte analog Beispiel 1.
Dabei wurden folgende Zwischenstufen isoliert und charakterisiert:
a) 2-(But-1-en-4-yl-amino)-benzoesäureamid (Formel III. R⁶ = -(CH₂)₂CH=CH₂, A = Benzoring mit R⁵ = Wasserstoff).
   Dargestellt aus 2-Aminobenzamid und 4-Brom-1-buten. Ausbeute: 59%.
   Farblose Kristalle. C₁₁H₁₄N₂O (190,2). F.: 114-115°C (Ethanol).
   IR (υ in cm-1): 1622 (C=O), 2930 (CH₂), 2942 (CH₂).
   MS m/e (%B): M^{+.} 190(15), 149(46), 132(100).
b) 1-(But-1-en-4-yl)-chinazolin-4(3H)-on-2(1H)-thion (Formel IV, R⁶ = -(CH₂)₂CH=CH_{2,} A = Benzoring mit R⁵ = Wasserstoff).
   Dargestellt aus 2-(But-1-en-4-yl)-aminobenzoesäureamid und Benzoylisothiocyanat in abs. acetonischer Lösung. Ausbeute: 39%.
   Hellgelbe Kristalle. C₁₂H₁₂N₂OS (232,3). F.: 184-185°C (Ethanol).
   IR (υ in cm⁻¹): 1608 (NH), 1682 (C=O), 2950 (CH₂).
   MS m/e (%B): M^{+.} 232(35), 203(74), 178(53), 162(8), 145(12), 132(29) 120(100).
c) (R,S)-3-Methyl-2,3-dihydro-1H,6H-[1,3]thiazino[3,2-a]chinazolin-6-on (Formel VI, R¹¹ = R¹² = Wasserstoff, R¹³ = Methyl, A = Benzoring mit R⁵ = Wasserstoff).

Dargestellt aus 1-(But-1-en-4-yl)-chinazolin-4(3H)-on-2(1H)-thion und konz. Bromwasserstoffsäure.
Der nach dem Erkalten gebildete Niederschlag wird bei Raumtemperatur mit 5%iger Natriumcarbonatlösung digeriert. Ausbeute: 84%.
Farblose Kristalle. C₁₂H₁₂N₂OS (232,3). F.: 198-201°C (Ethanol).
IR (υ in cm⁻¹): 1636 (C=N), 1708 (C=O), 2926 (CH₂), 2962 (CH₂).
MS m/e (%B): M^{+.} 232(100), 204(77), 162(36), 132(42).
UV_{Ethanol} (in nm)(log ε): 230.5 (4.27), 257.5 (4.46), 305.5 (3.94).

Die Umsetzung der nach c) gewonnenen Verbindung analog der unter Beispiel 1 d) aufgeführten Verfahrensweise ergibt (R,S)-1-(3-Mercaptobut-1-yl)-chinazolin-2,4(1H,3H)-dion.

### Beispiel 4

1-(3-Mercaptopropyl)-chinazolin-2,4(1 H,3H)-dion
(Formel la, R¹ = R² = R⁴ = Wasserstoff, R³ = Mercapto, n = 2,
A = Benzoring mit R⁵ = Wasserstoff).
a) 2,3-Dihydro-1H,6H-[1,3]thiazino[3,2-a]chinazolin-6-on (Formel VI, R¹¹ = R¹² = R¹³ = Wasserstoff, A = Benzoring mit R⁵ = Wasserstoff).
   5,04 g (24 mmol) 2-Ammoniumbenzoesäuremethylesterthiocyanat (Formel IX, A = Benzoring mit R⁵ = Wasserstoff) werden mit 50,0 g 1,3-Dibrompropan unter ständigem Rühren 150 Minuten am Rückflußkühler erhitzt. Nach dem Erkalten des Reaktionsansatzes wird der gebildete Niederschlag abgesaugt, mit Diethylether gewaschen und getrocknet. Das Rohprodukt wird in Wasser gelöst und die Lösung filtriert. Durch Zugabe von 10%iger Natronlauge (bis pH 10) zum Filtrat wird ein Niederschlag gewonnen, der abgefrittet und mit Wasser gewaschen wird. Nach dem Trocknen des Festsoffes wird dieser mit 30 ml Chloroform intensiv geschüttelt. Der unlösliche Rückstand wird isoliert und nach dem Trocknen umkristallisiert.
   Farblose Kristalle. C₁₁H₁₁N₂OS (218,3). F.: 236°C (Methylglykol).
   Ausbeute: 27% (bezogen auf 2-Ammoniumbenzoesäuremethylesterthiocyanat).
   IR (υ in cm⁻¹): 1600 (C=N), 1632 (C=O), 2926 (CH₂), 2956 (CH₂).
   MS m/e (%B): M^{+.} 218(100), 190(96), 162(25), 132(46), 118(14).
   UV_{Ethanol} (in nm)(log ε): 230.4 (4.23), 257.4 (4.37), 305,0 (3.88), 314,6 (3,84).
b) Aus 1,0 g (4,6 mmol) der nach a) erhaltenen Verbindung wird analog der unter Beispiel 1d) aufgeführten Verfahrensweise 1-(3-Mercaptopropyl)-chinazolin-2,4(1 H,3H)-dion gewonnen.
   Farblose Kristalle. C₁₁H₁₂N₂O₂S (236,3). F.: 154-157°C (Ethanol).
   Ausbeute: 79%.
   IR (υ in cm-1): 1610 (NH), 1682 (C=O), 1702 (CH₂), 2544 (SH).
   MS m/e (%B): M^{+.} 236(81), 203(100), 189(10), 176(32), 162(16), 160(26), 146(33), 132(98).
   UV_{Ethanol} (in nm)(log ε): 220.4(4.74), 245.2 (4.13), 313.0 (3.87).

### Beispiel 5

1-(2-Mercaptoethyl)-chinazolin-2,4(1H,3H)-dion
(Formel la, R¹ = R² = R⁴ = Wasserstoff, R³ = Mercapto, n = 1,
A = Benzoring mit R⁵ = Wasserstoff).
a) 1,2-Dihydro-5H-thiazolo[3,2-a]chinazolin-5-on (Formel V, R⁷ = R⁸ = R⁹ = R¹⁰ Wasserstoff, A = Benzoring mit R⁵ = Wasserstoff).
   Die Reindarstellung dieser literaturbekannten Verbindung erfolgt analog Beipiel 4 nach der unter a) angegebenen Verfahrensweise aus 5,04 g (24 mmol) 2-Ammoniumbenzoe- säuremethylesterthiocyanat und 50,0 g 1,2-Dibromethan. Farblose Kristalle.
   C₁₀H₈N₂OS (204,3). F.: 238-239°C (Methylglykol).
   Ausbeute: 31% (bezogen auf 2-Ammoniumbenzoesäuremethylesterthiocyanat).
   IR (υ in cm-1): 1608 (C=N), 1644 (C=O), 2948 (CH₂), 2986 (CH₂).
   MS m/e (%B): M^{+.} 204(100), 176(83), 132(38), 104(11).
   UV_{Ethanol} (in nm)(log ε): 232.8 (4.13), 254.4 (4.21), 308,8 (3.63).
b) Aus 1,02 g (5,0 mmol) der nach a) erhaltenen Verbindung wird analog der unter Beispiel 1 d) aufgeführten Verfahrensweise 1-(2-Mercaptoethyl)-chinazolin-2,4(1 H,3H)-dion gewonnen.
   Farblose Kristalle. C₁₀H₁₀N₂O₂S (222,3). F.: 178-181°C (Ethanol).
   Ausbeute: 84%.
   IR (υ in cm-1): 1606 (NH), 1698 asymm. (C=O), 2552 (SH).
   MS m/e (%B): M^{+.} +1 222(100).
   UV_{Ethanol} (in nm)(log ε): 220.4 (4.43), 245.2 (3.83), 313.0 (3.57).

### Beispiel 6

1-(2-Mercaptoethyl)-3-methyl-chinazolin-2,4(1H,3H)-dion
(Formel Ia, R¹ = R² = Wasserstoff, R³ = Mercapto, R⁴ = Methyl. n = 1,
A = Benzoring mit R⁵ = Wasserstoff).

1,92 g (10 mmol) des literaturbekannten 3-Methyl-chinazolin-4(3H)-on-2(1H)-thion (Formel VII, R⁴ = Methyl, A = Benzoring mit R⁵ = Wasserstoff) werden in 30 ml abs. Dimethylformamid eingetragen und mit 1,5 g (11 mmol) getrocknetem Kaliumcarbonat versetzt. Unter Rühren werden dem Ansatz 2,23 g (12 mmol) 1,2-Dibromethan zugetropft. Nach 24 Stunden wird der Reaktionsansatz mit 150 ml 10%iger Salzsäure versetzt und 8 Stunden am Rückflußkühler erhitzt und anschließend heiß filtriert. Nach dem Erkalten wird der Ansatz 12 Stunden bei ca. 4 °C aufbewahrt. Der gebildete Niederschlag wird mit Wasser gewaschen und nach dem Trocknen umkristallisiert. Es werden 780 mg dc-reines 1-(2-Mercaptoethyl)-3-methyl-chinazolin-2,4(1H,3H)-dion erhalten. Farblose Kristalle.
C₁₁H₁₂N₂O₂S (236,3). F.: 171-175°C (Ethanol).
Ausbeute: 33%.
IR (υ in cm-1): 1648 (C=O), 1696 (C=O), 2560 (SH), 2942 (CH₂).
MS m/e (%B): M^{+.} 236(13), 189(6), 176(100), 146(7), 132(81), 119(34).
UV_{Ethanol} (in nm)(log ε): 220.5 (4.60), 248.0 (3.88), 311,0 (3.68).

### Beispiel 7

1-(3-Mercaptoprop-1-yl)-3-methyl-chinazolin-2,4(1H,3H)-dion
(Formel la, R¹ = R² = Wasserstoff, R³ = Mercapto, R⁴ = Methyl, n = 2,
A = Benzoring mit R⁵ = Wasserstoff).

Analog Beispiel 6 werden 1,92 g (10 mmol) 3-Methyl-chinazolin-4(3H)-on-2(1H)-thion mit 2,42 g (12 mmol) 1,3-Dibrompropan umgesetzt. Es werden 525 mg dc-reines 1-(3-Mercaptoprop-1-yl)-3-methyl-chinazolin-2,4(1H,3H)-dion erhalten. Farblose Kristalle.
C₁₂H₁₄N₂O₂S (250,3). F.: 105-107°C (Ethanol). Ausbeute: 21%.
IR (υ in cm⁻¹): 1640 (C=O), 1700 (C=O), 2542 (SH), 2932 (CH₂).
MS m/e (%B): M^{+.} 250(65), 217(83), 203(9), 190(30), 176(13), 160(27), 146(35).

### Beispiel 8

3-Ethyl-1-(2-mercaptoethyl)-chinazolin-2,4(1H,3H)-dion
(Formel la, R¹ = R² = Wasserstoff, R³ = Mercapto, R⁴ = Ethyl, n = 1,
A = Benzoring mit R⁵ = Wasserstoff).

Analog Beispiel 6 werden 2,06 g (10 mmol) des literaturbekannten 3-Ethylchinazolin-4(3H)-on-2(1H)-thion mit 2,23 g (12 mmol) 1,2-Dibromethan umgesetzt. Es werden 475 mg dc-reines 3-Ethyl-1-(2-mercaptoethyl)-chinazolin-2,4(1H,3H)-dion erhalten. Farblose Kristalle.
C₁₂H₁₄N₂O₂S (250,3). F.: 114-115°C (Ethanol). Ausbeute: 19%.
IR (υ in cm-1): 1660 (C=O), 1696 (C=O), 2569 (SH).
MS m/e (%B): M^{+.} 250(11), 203(6), 190(64), 162(41), 146(16), 132(100).

### Beispiel 9

3-Ethyl-1-(3-mercaptoprop-1-yl)-chinazolin-2,4(1 H,3H)-dion
(Formel la, R¹ = R² = Wasserstoff, R³ = Mercapto, R⁴ = Ethyl, n = 2, A = Benzolring mit R⁵ = Wasserstoff).

Analog Beispiel 6 werden 2,06 g (10 mmol) 3-Ethyl-chinazolin-4(3H)-on-2(1H)-thion mit 2,42 g (12 mmol) 1,3-Dibrompropan umgesetzt. Es werden 607 mg dc-reines 3-Ethyl-1-(3-mercaptoprop-1-yl)-chinazolin-2,4(1H,3H)-dion erhalten. Farblose Kristalle.
C₁₃H₁₆N₂O₂S × H₂O (264,3 × 18). F.: 110-114°C (Ethanol). Ausbeute: 23%.
IR (υ in cm-1): 1652 (C=O), 1702 (C=O), 2562 (SH), 2932 (CH₂).
MS m/e (%B): M^{+.} 264(55), 231(67), 217(8), 203(16), 176(42), 160(23), 146(45).
UV_{Ethanol} (in nm)(log ε): 222.2 (4.75), 244.0 (4.08), 313,0 (3.87).

### Beispiel 10

6,7-Dimethoxy-1-(2-mercaptoethyl)-3-methyl-chinazolin-2,4(1H,3H)-dion
(Formel Ia, R¹ = R² = Wasserstoff, R³ = Mercapto, R⁴ = Methyl, n = 1, A = Benzoring mit R⁵ in 6- und 7-Position jeweils -OCH₃).

Analog Beispiel 6 werden 2,52 g (10 mmol) des literaturbekannten 6,7-Dimethoxy-3-methyl-chinazolin-4(3H)-on-2(1H)-thion mit 2,23 g (12 mmol) 1,2-Dibromethan umgesetzt. Es werden 1,36 g dc-reines 6,7-Dimethoxy-1-(2-mercaptoethyl)-3-methyl-chinazolin-2,4(1H,3H)-dion erhalten. Farblose Kristalle.
C₁₃H₁₆N₂O₄S (296,3). F.: 121-122°C (Ethanol). Ausbeute: 46%.
IR (υ in cm-1): 1650 (C=O), 1696 (C=O), 2550 (SH), 2936 (CH₂).
MS m/e (%B): M^{+.} 296(52), 236(100), 221(43), 192(89), 164(18), 149(20), 134(13).
UV_{Ethanol} (in nm)(log ε): 237.0 (4.59), 259.5 (3.95), 322.0 (3.84).

### Beispiel 11

3-Benzyl-1-(2-mercaptoethyl)-chinazolin-2,4(1H,3H)-dion
(Formel Ia, R¹ = R² = Wasserstoff, R³ = Mercapto, R⁴ = Benzyl, n = 1,
A = Benzoring mit R⁵ = Wasserstoff).

Analog Beispiel 6 werden 2,68 g (10 mmol) des literaturbekannten 3-Benzylchinazolin-4(3H)-on-2(1H)-thion mit 2,23 g (12 mmol) 1,2-Dibromethan umgesetzt. Es werden 905 mg dc-reines 3-Benzyl-1-(2-mercaptoethyl)-chinazolin-2,4(1H,3H)-dion erhalten.
Farblose Kristalle.
C₁₇H₁₆N₂O₂S (312,4). F.: 111-114°C (Ethanol). Ausbeute: 29%.
IR (υ in cm-1): 1656 (C=O), 1698 (C=O), 2573 (SH).
MS m/e (%B): M^{+.} 312(13), 252(93), 235(7), 146(23), 132(100).
UV_{Ethanol} (in nm)(log ε): 223.0 (4.81), 246.2 (4.15), 311,8 (3.84).

### Beispiel 12

3-(4-Mercaptobut-1-yl)-chinazolin-2,4-(1 H,3H)-dion
(Formel Ib, Alk* = Butylen (-(CH₂)₄-), X = Mercapto,
A = Benzoring mit R⁵ = Wasserstoff).
a) 3-(4-Hydroxybut-1-yl)-chinazolin-4(3H)-on-2(1H)-thion (Formel XI, A = Benzoring mit R⁵ = Wasserstoff)
   9,65 g (0,05mol) des literaturbekannten 2-lsothiocyanatobenzoesäuremethylester (Formel X, Alk Methyl und A = Benzoring mit R⁵ = Wasserstoff) werden zu 4,45 g (0,05mol) 4-Aminobutan-1-ol gegeben. Die Reaktionsmischung wird 24 Stunden bei Raumtemperatur gerührt. Anschließend werden 40 ml Wasser hinzugefügt. Der gebildete Niederschlag wird abgefrittet, mit Wasser gewaschen und nach dem Trocknen umkristallisiert. Es werden 10,6 g 3-(4-Hydroxybut-1-yl)-chinazolin-4(3H)-on-2(1H)-thion erhalten.
   Farblose Kristalle.
   C₁₂H₁₄N₂O₂S × 0.5 H₂O (250,3 + 9,0). F.: 168-172°C (Ethanol).
   Ausbeute: 85%.
   IR (υ in cm-1): 1622 (NH), 1652 (C=O), 2950 (CH₂), 3408 (OH).
   MS m/e (%B): M^{+.} +1 251(100).
b) 2,3,4,5-Tetrahydro-7H-[1,3]thiazepino[2,3-b]chinazolin-7-on (Formel XII. A = Benzoring mit R⁵ = Wasserstoff)
   5,0 g (0,02mol) der nach a) hergestellten dc-reinen Verbindung werden in 35 ml konz. Salzsäure eingetragen. Der Ansatz wird 90 Minuten am Rückflußkühler erhitzt. Nach dem Erkalten wird der Niederschlag abgesaugt. Anschließend wird unter Rühren zu dem Kristallisat bis pH 8 5%ige Natriumcarbonat-Lösung hinzugefügt. Der gesammelte Niederschlag wird mit Wasser gewaschen und nach dem Trocknen umkristallisiert. Es werden 3,8 g 2,3,4,5-Tetrahydro-7H-[1,3]thiazepino[2,3-b]chinazolin-7-on erhalten.
   Farblose Kristalle.
   C₁₂H₁₂N₂OS (232,3). F.: 116-118°C (Ethanol).
   Ausbeute: 82%.
   IR (υ in cm-1): 1604 (C=N), 1668 (C=O), 2960 (CH₂).
   MS m/e (%B): M^{+.} 232(31), 217(100), 199(54), 179(34), 162(69), 146(30), 119(43).
   UV_{Ethanol} (in nm)(log ε): 226.0 (4.38), 295.5 (4.16).
c) 3-(4-Mercaptobut-1-yl)-chinazolin-2,4(1 H,3H)-dion
   (Formel Ib, Alk* = n-Butylen (-(CH₂)₄-), X = Mercapto,
   A = Benzoring mit R⁵ = Wasserstoff)
   1,50 g (6,45 mmol) der nach b) hergestellten Verbindung werden in eine aus 0,6 mi konzentrierter Schwefelsäure, 1,5 ml Eisessig und 66 ml Wasser bereitete Säuremischung eingetragen und am Rückflußkühler 8 Stunden erhitzt. Der nach dem Erkalten gebildete Niederschlag wird abgefrittet, mit wenig Wasser gewaschen und nach dem Trocknen umkristallisiert. Es werden 1,43 g 3-(4-Mercaptobut-1-yl)-chinazolin-2,4(1H,3H)-dion erhalten.
   Farblose Kristalle.
   C₁₂H₁₄N₂O₂S (250,3). F.: 157-160°C (Ethanol).
   Ausbeute: 89%.
   IR (υ in cm⁻¹): 1606 (NH), 1664 (C=O), 1712 (C=0), 2573 (SH), 2936 (CH₂).
   MS m/e (%B): M^{+.} 250(11), 217(51), 203(3), 187(4), 163(100), 146(76), 119(35).
   UV_{Ethanol} (in nm)(log ε): 219,5 (4.67), 244,0 (3.97), 310.5 (3.65).

### Beispiel 13

3-(5-Mercaptopent-1-yl)-chinazolin-2.4(1H,3H)-dion
(Formel Ib, Alk* = Pentylen (-(CH₂)₅-), X = Mercapto,
A = Benzoring mit R⁵ = Wasserstoff).
a) 3-(5-Hydroxypent-1-yl)-chinazolin-2,4(1 H,3H)-dion
   (Formel XV, Alk* = Pentylen (-(CH₂)₅-), A = Benzoring mit R⁵ = Wasserstoff)
   6,27 g (0,03mol) des literaturbekannten 2-Methoxycarbonylaminobenzoesäuremethylester (Formel XIII, Alk = jeweils Methyl, A = Benzoring mit R⁵ = Wasserstorf) werden mit 5,15 g (0,05mol) 5-Aminopentan-1-ol (Formel XIV, Alk* = Pentylen (-(CH₂)₅-)) 10 Minuten unter mäßiger Wärmezufuhr am Rückflußkühler erhitzt. Nach Erkalten des Ansatzes werden unter Rühren 100 ml Wasser und anschließend bis ca. pH 4 verdünnte Salzsäure hinzugefügt. Nach einiger Zeit kristallisiert nach Zugabe von wenig Ethanol das Rohprodukt aus. Der gebildete Niederschlag wird abgefrittet und mit Wasser gewaschen. Nach dem Trocknen werden
   6,18 g 3-(5-Hydroxypent-1-yl)-chinazolin-2,4(1H,3H)-dion erhalten.
   Farblose Kristalle.
   C₁₃H₁₆N₂O₃ x H₂O (248,3 + 18,0). F.: 125-129°C (Ethanol/Wasser).
   Ausbeute: 83%.
   IR (υ in cm⁻¹): 1632 asymm. (NH, C=O), 1720 (C=0), 2936 (CH₂).
   MS m/e (%B): M^{+.} 248(10), 231(8), 218(20), 176(35), 163(100), 146(77), 119(56).
b) 3-(5-Brompent-1-yl)-chinazolin-2,4(1H,3H)-dion
   (Formel XVI, Alk* = Pentylen (-(CH₂)₅-), Hal = Brom, A = Benzoring mit R₅ = Wasserstoff)
   2,48 g (0.01 mol) der nach a) hergestellten dc-reinen Verbindung werden in 25 ml konz. Bromwasserstoffsäure eingetragen. Der Ansatz wird 30 Minuten am Rückflußkühler erhitzt. Nach dem Erkalten wird der Niederschlag abgesaugt, mit Wasser neutral gewaschen und getrocknet. Es werden 2,89 g dc-reines 3-(5-Brompent-1-yl)-chinazolin-2,4(1 H,3H)-dion erhalten.
   Farblose Nadeln.
   C₁₃H₁₅N₂O₂Br (311,2). F.: 169-172°C (Ethanol).
   Ausbeute: 93%,
   IR (υ in cm-1): 1622 (NH), 1666 (C=O), 1712 (C=O), 2940 (CH₂).
   MS m/e (%B): M^{+.} +1 313(96), 311(100).
c) 3-(5-Mercaptopent-1-yl)-chinazolin-2,4(1 H,3H)-dion
   (Formel Ib, Alk* = n-Pentylen (-(CH₂)₅-), X = Mercapto,
   A = Benzoring mit R₅ = Wasserstoff)
   1,55 g (5,0 mmol) der nach b) hergestellten Verbindung werden in 5 ml Methylglykol eingetragen. Es werden 0,46 g (6 mmol) Thioharnstoff hinzugefügt. Der Ansatz wird 30 Minuten am Rückflußkühler erhitzt. Nach dem Abkühlen werden dem Reaktionsansatz 50 ml Wasser und 4%ige Natronlauge bis zum Erreichen einer alkalischen Reaktion hinzugefügt. Nach dem Aufklaren wird die Lösung filtriert und mit 10%iger Salzsäure angesäuert. Der ausgefallene Niederschlag wird wird gewaschen und getrocknet. Es werden 780 mg 3-(5-Mercaptopent-1-yl)-chinazolin-2,4(1H,3H)-dion erhalten.
   Farblose Kristalle.
   C₁₃H₁₆N₂O₂S (264,3). F.: 137-138°C (Ethanol).
   Ausbeute: 59%.
   IR (υ in cm-1): 1636 asymm. (NH, C=O), 1726 (C=O), 2585 (SH), 2936 (CH₂).
   MS m/e (%B): M^{+.} 264(9), 231(46), 176(10), 163(100), 146(48), 119(26).
   UV_{Ethanol} (in nm)(log ε): 220.0 (4.91), 243.5 (4.23), 310.8 (3.93).

### Beispiel 14

### 1 -(3-Mercaptoprop-1-yl)-3-methyl-thieno[32-d]-pyrimidin-24(1 H,3H)-dion Formel la, R¹=R²=Wasserstoff, R³=Mercapto, R⁴=Methyl, n=2, A= in 2,3-Position anellierter Thiophenring

Analog Beispiel 6 werden 0,99 g (5 mmol) des literaturbekannten 3-Methylthieno[3.2-d]-pyrimidin-4(3H)on-2(1H)-thion mit 1,11 g (5,5 mmol) 1,3-Diprompropan und 0,75 g (5,4 mmol) Kaliumcarbonat in 15 ml abs. Dimethylformamid umgesetzt. Nach 2 h wird der Reaktionsansatz mit 60 ml 10 %iger Salzsäure versetzt und 3 h unter Rückfluß erhitzt. Es werden 1,14 g dc-reines 1-(3-Mercaptopropyl-1-yl)-3-methyl-thieno[3.2-d]-pyrimidin-2,4(1 H,3H)-dion erhalten.
Ockerfarbene Kristalle
F.: 174-176°C (Ethanol)
Ausbeute 89%
C₁₀H₁₂N₂O₂S₂ (256,3)
MS m/e (%B): M^{+.} 256(100), 223(50), 196(39), 152(40), 138(93), 125(23), 110(24). IR ((ν in cm⁻¹): 1660 (C=O), 1691 (C=O), 2560 (SH)
UV (in mm) (log ε): 219,6(0,42), 273,7(3,23), 306,2(3,42)

### Beispiel 15

Tabletten mit 50,0 mg 1-(3-Mercaptoprop-1-yl)-3-methyl-chinazolin-2,4(1H,3H)-dion

### Zusammensetzung:

1 Tablette enthält 50,0 mg Wirkstoff, 32,0 mg Mikrokristalline Cellulose, 20,0 mg Lactose, 15,0 mg Kartoffelstärke, 8,0 mg Talkum, 3,5 mg Polyvinylpyrrolidon und 1,5 mg Magnesiumstearat.

### Herstellungsverfahren:

Der gepulverte Wirkstoff wird mit Mikrokristalline Cellulose, Lactose und Kartoffelstärke vermischt und mit einer 20%igen ethanolischen Lösung des Polyvinylpyrrolidinons gleichmäßig durchfeuchtet, durch ein Sieb der Maschenweite 1,5 mm gedrückt, bei 40 °C getrocknet und erneut durch ein Sieb (Maschenweite 1,0 mm) gepreßt. Das erhaltene Granulat wird mit Talkum und Magnesiumstearat gemischt und zu Tabletten verpreßt.
Tablettenmasse: 130 mg.

### Beispiel 16

Dragees mit 35,0 mg 1-(3-Mercaptoprop-1-yl)-chinazolin-2,4(1H,3H)-dion

### Zusammensetzung:

1 Drageekern enthält 35,0 mg Wirkstoff, 20,0 mg Mikrokristalline Cellulose, 10,0 mg Lactose, 16,5 mg Maisstärke, 5,0 mg Talkum, 2,8 mg Polyvinylpyrrolidon und 0,7 mg Magnesiumstearat.

### Herstellungsverfahren:

Die mit Mikrokristalline Cellulose, Lactose und Kartoffelstärke vermischte pulverförmige Wirksubstanz wird mit einer 20%igen ethanolischen Lösung des Polyvinyipyrrolidinons gleichmäßig durchfeuchtet, durch ein Sieb der Maschenweite 1,5 mm gedrückt, bei 40 °C getrocknet und nochmals durch ein Sieb (Maschenweite 1,0 mm) gepreßt. Das erhaltene Granulat wird mit Talkum und Magnesiumstearat gemischt und zu Drageekernen verpreßt.
Kernmasse: 90mg.

Die hergestellten Drageekerne werden nach üblichen Verfahren mit einer Hülle überzogen und auf übliche Weise poliert.

### Beispiel 17

### 1-(2-Mercaptoethyl)-3-methyl-thieno[3,2-d]pyrimidin-2,4(1H,3H)-dion Formel la, R¹=R²=Wasserstoff, R³=Mercapto, R⁴=Methyl, n=1, A= in 2,3-Position anellierter Thiophenring

Analog Beispiel 16 werden 0,99 g (5 mmol) des literaturbekannten 3-Methylthieno[3,2-d]-pyrimidin-4(3H)on-2(1H)-thion mit 1,03 g (5,5 mmol) 1,2-Dipromethan umgesetzt. Es werden 420 mg dc-reines 1-(2-Mercaptoethyl)-3-methyl-thieno[3,2-d] pyrimidin-2,4(1 H,3H)-dion erhalten.
Ockerfarbene Kristalle
F.: 158-160°C (Ethanol)
Ausbeute: 41%
C₉H₁₀N₂O₂S₂ (242,3)
IR ((ν in cm⁻¹): 1648 (C=O), 1692 (C=O), 2590 (SH)
MS m/e (%B): M^{+.} 242(27), 182(83), 138(100), 125(32), 110(18).
UV (in mm) (log ε): 222,5(4,20), 270,8(2,78), 316,7(3.05)

### Beispiel 18

### (R,S)-3-(3-(1-Methyl)-mercaptopropyl)-chinazolin-2,4(1H,3H)-dion Formel Ib, Alk*= 1-Methyl-propyl, X= 3-Mercapto, A= anellierter Benzoring mit R⁵=H

### a.) 2-Crotylthiochinazolin-4(3H)-on

Die literaturbekannte Verbindung wurde aus 3.56 g (20 mmol) Chinazolin-4(3H)-on-2(1H)-thion und 1,81 g (20 mmol) Crotylchlorid dc-rein hergestellt.
Farblose Kristalle
F.: 193-194°C (Ethanol)
Ausbeute 71%
C₁₂H₁₂N₂OS (232,3)
IR ((ν in cm⁻¹): 1613 (C=N), 1673 (C=O), 2953 (CH₂)
MS m/e (%B): M⁺ +1 233(100)
UV (in mm) (log ε): 230,5(4,47), 274,5(4,24), 314,5(3,84)

### b.) (R,S)-4-Methyl-3,4-dihydro-2H,6H-[1,3]thiazino[2,3-b]chinazolin-6-on

1,15 g (5 mmol) der unter a.) erhaltenen Verbindung werden in 5 ml konzentrierter H₂SO₄ eingetragen und bei 40°C für 2 h gerührt. Die Lösung wird auf ca. 100 ml Eiswasser gegeben und mit gesättigter Na₂CO₃-Lösung, ggf. mit 1N NaOH neutralisiert. Der ausgefallene Niederschlag wird abgesaugt, mit H₂O gewaschen und umkristallisiert.
Farblose Kristalle
F.: 111-112°C (Ethanol)
Ausbeute 51%
C₁₂H₁₂N₂OS (232,3)
IR ((ν in cm⁻¹): 1608 (C=N), 1678 (C=O), 2938 (CH₂)
MS m/e (%B): M⁺ 232(100)
UV (in mm) (log ε): 235,0(4,32), 283,8(4,15)

### c.) (R,S)-3-(3-(1-Methyl)-mercaptopropyl)-chinazolin-2,4(1H,3H)-dion

Formel Ib, Alk*= 1-Methyl-propyl, X= 3-Mercapto, A= anellierter Benzoring mit R⁵=H
Analog Beispiel 1, Schritt 1.d.) werden 0,5 g (2 mmol) Thiazinoverbindung b.) in das Säuregemisch eingetragen, zurückfließend erhitzt und anschließend umkristallisiert.
Farblose Kristalle
F.: 114-118°C (Ethanol)
Ausbeute: 71%
C₁₂H₁₂N₂O₂S (250,3)
IR ((ν in cm⁻¹): 1620 (NH), 1644 (C=O), 1720 (C=O), 2562 (SH)
MS m/e (%B): M⁺ 250(11), 232(13), 217(96), 201(14), 163(100), 146(79), 119(26), 90(29)
UV (in mm) (log ε): 220,0(4,67), 242,0(4,10), 313,0(3,87)

### Beispiel 19

### (R,S)-3-(3-(2-Methyl)-mercaptopropyl)-chinazolin-2,4(1H,3H)-dion

Formel Ib, Alk*= 2-Methyl-propyl, X= 3-Mercapto, A= anellierter Benzoring mit R⁵=H

### a.) (R,S)-3-Methyl-3,4-dihydro-2H,6H-[1,3]thiazino[2,3-b]chinazolin-6-on

3,56 g (20 mmol) der literaturbekannten Verbindung Chinazolin-4(3H)-on-2(1H)thion und 2,6 g (20 mmol) K₂CO₃ werden in 30 ml Dimethylformamid eingetragen und nach Lösung des Chinazolins mit 2,19 g (22 mmol) 1-Chlor-3-brom-2-methylpropan 2 Tage geschüttelt, der Niederschlag abgesaugt und umkristallisiert.
Farblose Kristalle
F.: 141-146°C (Ethanol)
Ausbeute 79%
C₁₂H₁₂N₂OS x H₂O (232,3)
IR ((ν in cm⁻¹): 1606 (C=N), 1682 (C=O), 2932 (CH₂)
MS m/e (%B): M⁺ 232(100), 217(38), 199(26), 162(46)
UV (in mm) (log ε): 237,0(4,28), 290,5(4,25)

### b.) (R,S)-3-(3-(2-Methyl)-mercaptopropyl)-chinazolin-2,4(1H,3H)-dion

### Formel Ib, Alk*= 2-Methyl-propyl, X= 3-Mercapto, A= anellierter Benzoring mit R⁵=H

Analog Beispiel 1, Schritt 1 d.) werden 0,5 g der unter a.) erhaltenen Verbindung umgesetzt.
Farblose Kristalle
F.: 194-197°C (Ethanol)
Ausbeute 79%
C₁₂H₁₂N₂O₂S (250,3)
IR ((ν in cm⁻¹): 1640 asymm. (NH, C=O), 1722 (C=O), 2572 (SH)
MS m/e (%B): M⁺ 250(24), 217(100), 201(17), 176(43), 163(57), 146(84), 119(47), 90(25)
UV (in mm) (log ε): 219,5(4,66), 240,0(4,12), 293,0(3,99)

### Beispiel 20

### N-Hydroxy-2-[3-(methyl-2.4-dioxo-3,4-dihydro-2H-chinazolin-1-yl)-propylsulfanyl]acetamid

### (Formel Ia, R¹=R²= Wasserstoff, R³= Hydroxycarbamoylmethylenthio, R⁴= Methyl, n= 2, A= Benzoring mit R⁵= Wasserstoff)

250 mg (1mmol) 1-(3-Mercaptoprop-1-yl)-3-methyl-chinazolin-24(1H,3H)-dion (vergl. Bsp. 7) werden unter Stickstoff in 4 ml absolutem DMF gelöst. Anschließend werden 330 mg (3mmol) 2-Chlor-N-hydroxyacetamid und 0,83 ml (6mmol) Triethylamin zugegeben und die Lösung 3 Stunden unter Stickstoff gerührt. Nach Zugabe von 15 mi Wasser wird unter Eiskühlung mit Eisessig angesäuert und noch 2 Stunden bei 0°C gerührt. Der ausgefallene Feststoff wird abgetrennt, mit Wasser gewaschen, getrocknet und umkristallisiert.
Farblose Kristalle
F.: 146-148°C (Essigester/Petrolether)
Ausbeute: 66%
C₁₄H₁₇N₃O₄S (323,4)
IR ((ν in cm⁻¹): 1488 (C=O), 1646 (C=O), 1697 (C=O)3200-3500 (OH)
MS m/e (%B): M⁺ 323(5), 264(5), 250(50), 217(35)
UV (in mm) (log ε): 215,4(4,26), 302,1(3,04), 332,9(2,71)

## Patentansprüche

1. Mehrcyclische Pyrimidin-2,4(1H,3H)-dione mit funktionalisierten Alkylresten in 1-und/oder 3-Position der allgemeinen Formel la und Ib, 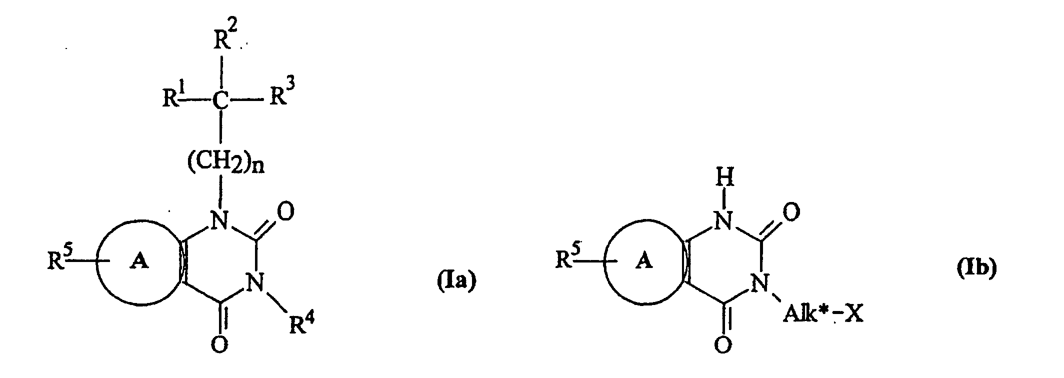 worin bedeuten:
R¹ Wasserstoff, Methyl, Ethyl,
R² Wasserstoff oder Methyl,
R³ Mercapto oder -SAlkCONHOH,
Alk verzweigtes oder unverzweigtes C₁-C₅ Alkyl en
R⁴ H, Alkyl, Benzyl sowie Phenyl,
n 0, 1 oder 2,
Alk* verzweigtes oder unverzweigtes C₄-C₁₂ Alkylen, mit Ausnahme von 3-Methylpropylen,
X Mercapto oder -SAlkCONHOH,
A anellierter Benzoring mit
R⁵ Wasserstoff, 6-Methyl, 8-Methyl, 6-Fluor, 6-Chlor, 6-Brom, 6-Methylthio oder 6,7-Dimethoxy oder
in 2,3-Position anellierter Thiophen-Ring,
der gegebenfalls in 4,5-Position mit Dimethyl substituiert oder mit einem Cyclopenten-, Cyclohexen oder Cyclohepten-Ring kondensiert ist,
einschließlich ihrer pharmazeutisch geeigneten Salze sowie Tautomeren.

2. Chinazolin-2,4(1H,3H)-dione mit funktionalisierten Alkylresten in 1- und/oder 3-Position nach Anspruch 1 der allgemeinen Formel IIa und IIb 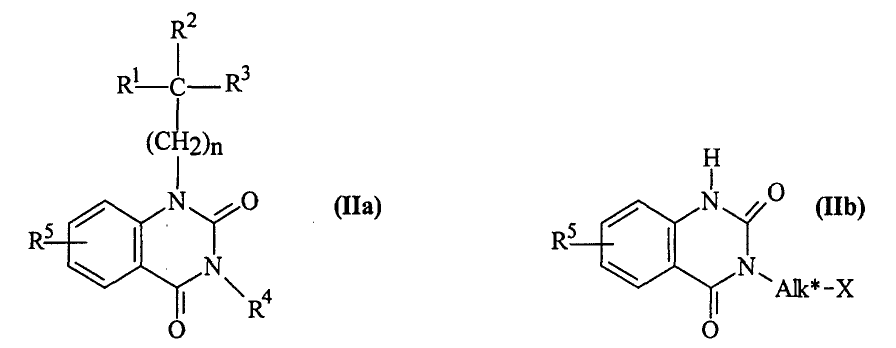 mit R¹, R², R³, R⁴, Alk, Alk*, n und X wie in Anspruch 1 einschließlich ihrer pharmazeutisch geeigneten Salze sowie ihre Tautomeren.

3. Verfahren zur Herstellung der Verbindung der allgemeinen Formel Ia nach Anspruch 1 mit R⁴ = H, **dadurch gekennzeichnet, dass** wie folgt verfahren wird:
- Umsetzung von 2-(Alkenylamino)-1-carbonsäureamiden der allgemeinen Formel III, 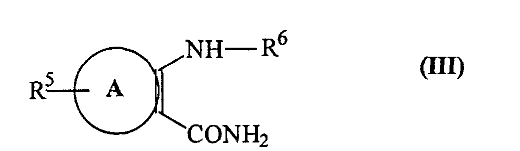 worin R₆ C₃-C₆ Alkenyl bedeutet und A wie in Anspruch 1 definiert ist, mit einem das C=S-Strukturelement übertragenden Agens in einem polaren, aprotischen Lösungsmittel,
- Rühren des Reaktionsansatzes,
- Abdestillieren des Lösungsmittels im Vakuum,
- Hinzufügen von verdünnter Alkalilauge und mäßiges Erwärmen bis etwa 60° C,
- Abtrennen ungelöster Bestandteile durch Filtration,
- Abkühlen und Ansäuern des Filtrates,
- Erhitzen der erhaltenen Verbindungen der allgemeinen Formel IV, 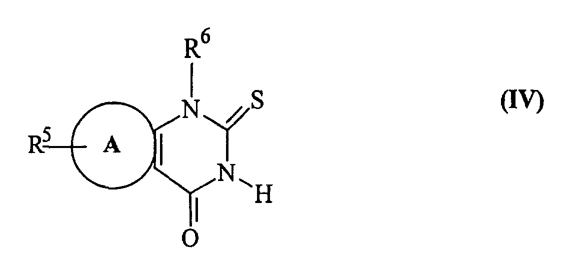
worin A, R⁵ und R⁶ dasselbe wie oben bedeuten,
- mit konzentrierter Mineralsäure oder Mischungen davon mit Eisessig und/oder Ameisensäure am Rückfluß,
- Abkühlen des Reaktionsansatzes,
- Trocknen der erhaltenen Verbindung der allgemeinen Formel V, 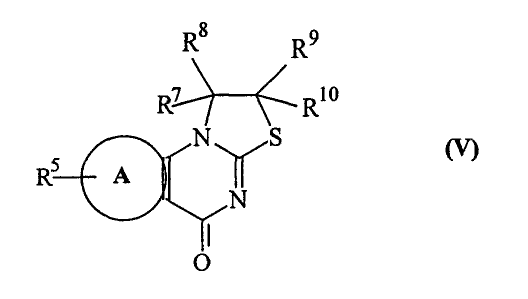 worin
R⁷, R⁸, R⁹ und R¹⁰ Wasserstoff, Methyl oder Ethyl und A und R⁵ dasselbe wie oben bedeuten,
- oder Trocknen der erhaltenen Verbindung der allgemeinen Formel VI, 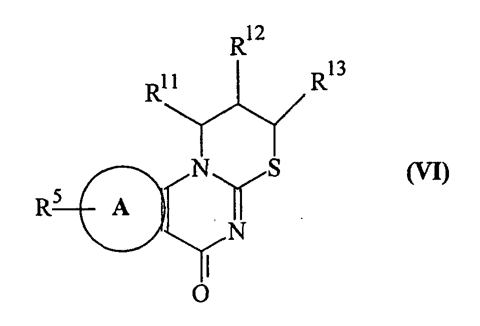 worin R¹¹, R¹², R¹³ Wasserstoff, Methyl oder Ethyl und A und R⁵ dasselbe wie oben bedeuten,
- in einem Vakuum-Exsikkator über Kaliumhydroxyd oder
- Einrühren der obigen Verbindungen in eine verdünnte, wässrige Natriumcarbonatlösung,
- Isolieren der Verbindungen,
- Erhitzen dieser Verbindungen in verdünnter Mineralsäure oder Mischungen davon mit Eisessig und/oder Ameisensäure bis zum Rückfluss,
- Abkühlen des Reaktionsansatzes,
- Waschen und Trocknen des Kristallisats unter Erhalt der Verbindung der allgemeinen Formel la oder ihrer Tautomeren, worin R⁴ ausnahmslos Wasserstoff bedeutet.

4. Verfahren zur Herstellung der Verbindungen der allgemeinen Formeln Ia nach Anspruch 1, worin R³ Mercapto bedeutet und R⁴, n, A, und R⁵ die Bedeutung wie in Anspruch 1 haben,
durch folgende Verfahrensschrittabfolge:
- Umsetzung von bi- und tricyclischen 3-Alkyl(bzw.Benzyl oder Phenyl)-pyrimidin-4(3H)-on-2(1H)-thionen der allgemeinen Formel VII, 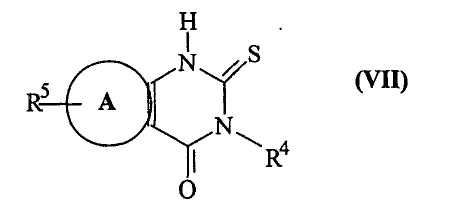 worin R⁴ H, Alk, Benzyl, Phenyl und Alk, A und R₅ dasselbe wie oben bedeuten, mit α,ω-Dihalogenalkanen der allgemeinen Formel VIII,
**Hal(CH**_{**2**}**)**_{**m**}**Hal (VIII)**
worin m 2, 3 oder 4 und Hal Chlor, Brom oder lod bedeuten,
- in einem aprotischen, dipolaren Lösungsmittel,
- Zugabe von verdünnter Salzsäure,
- längerem Erhitzen bis zum Rückfluß,
- Filtration der heißen Reaktionslösung,
- Abkühlen und Aufbewahren der Lösung bei 4° C
- unter Erhalt der Verbindungen der allgemeinen Formel la,
worin R¹ = R² = H, R³ Mercapto, R⁴ H, Alk, Benzyl, Phenyl und Alk, A und R⁵ dasselbe wie oben bedeuten,
oder
Umsetzung von 2-Ammoniumcarbonsäuremethylesterthiocyanaten der allgemeinen Formel IX, 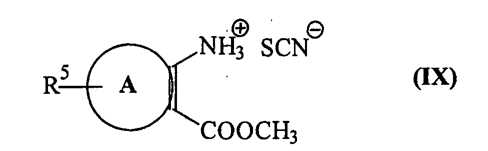 worin A und R⁵ dasselbe wie oben bedeuten,
mit α,ω-Dihalogenalkanen der allgemeinen Formel VIII,
worin m 2 oder 3 und Hal Chlor, Brom oder lod bedeuten,
- Erhitzen der Reaktionspartner unter Rühren bis zum Rückfluss,
- Abkühlen und Isolieren des Niederschlages,
- Waschen mit Diethylether und Trocknen,
- Lösen des Niederschlages in Wasser,
- Filtrieren der Lösung und Zugabe von verdünnter Natronlauge bis pH 10,
- Isolierung des ausgefallenen Niederschlages und Waschen mit Wasser,
- Trocknen des Niederschlages und intensives Schütteln mit Chloroform,
- Isolierung des Niederschlages und Trocknen,
- Umkristallisieren mit einem organischen Lösungsmittel,
unter Erhalt der Verbindungen der allgemeinen Formel V, 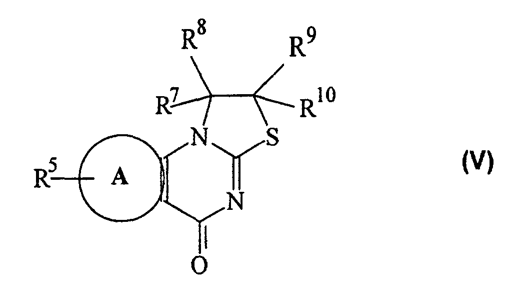 worin R⁷, R⁸, R⁹ und R¹⁰ Wasserstoff und A und R⁵ dasselbe wie oben bedeuten, gewonnen durch Umsetzung mit Dibrommethan,
oder der Verbindung der allgemeinen Formel VI, 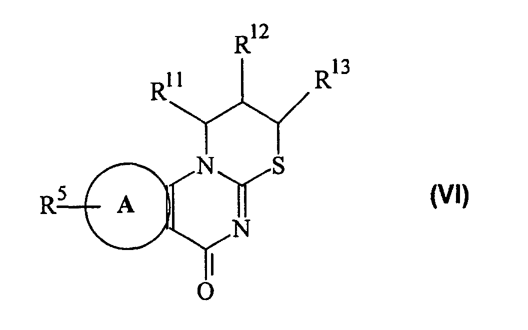 gewonnen durch Umsetzung mit 1,3-Dibrompropan, worin R¹¹, R¹², R¹³ Wasserstoff und A und R⁵ dasselbe wie oben bedeuten,
- Erhitzen der vorstehend genannten Verbindungen der allgemeinen Formel V bzw. VI in verdünnter Mineralsäure oder Mischungen davon mit Eisessig und/oder Ameisensäure bis zum Rückfluß,
- Abkühlen des Reaktionsansatzes,
- Waschen und Trocknen des Kristallisats unter Erhalt der Verbindung der allgemeinen Formel la oder ihrer Tautomeren,
worin R¹, R², R⁴ Wasserstoff, R³ Mercapto, n 1 oder 2 und A und R⁵ dasselbe wie in Anspruch 1 bedeuten.

5. Verfahren zur Herstellung der Verbindungen der allgemeinen Formel Ib nach Anspruch 1, worin Alk* n-Butylen, X Mercapto und A wie in Anspruch 1 bezeichnet bedeuten, **gekennzeichnet durch** die Umsetzung von 2-Isothiocyanato-1-carbonsäureester der allgemeinen Formel X, 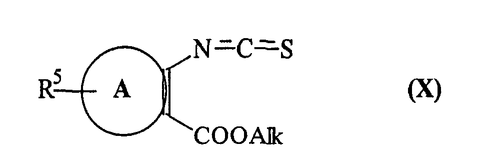 worin Alk, A und R⁵ dasselbe wie oben bedeuten,
- mit 4-Aminobutan-1-ol bei Raumtemperatur unter intensivem und längerem Rühren
- Hinzufügen von Wasser unter Erhalt der Verbindungen der allgemeinen Formel XI, 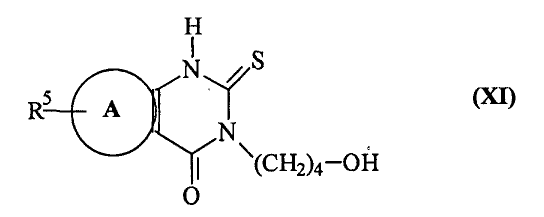 worin A und R⁵ dasselbe wie oben bedeuten,
- Umsetzung der Verbindungen der allgemeinen Formel XI mit konzentrierter Mineralsäure oder Mischungen davon mit Eisessig und/oder Ameisensäure bis zum Rückfluss,
- Abkühlen des Reaktionsansatzes und Isolieren des Kristallisates,
- Hinzufügen von wäßriger Natriumcarbonatlösung zum Kristallisat bis etwa pH 9,
- Isolieren des Kristallisates, Waschen mit Wasser und Trocknen unter Erhalt der mehrcyclischen 2,3,4,5-Tetrahydro-7H-[1,3]thiazepino[2,3-a]pyrimidin-7-one der allgemeinen Formel XII, 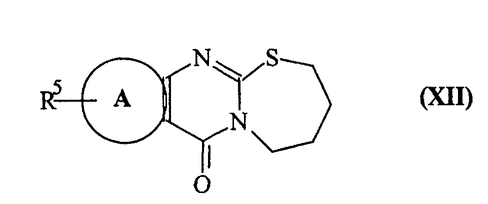 worin A und R⁵ dasselbe wie oben bedeuten.
- Erhitzen der vorstehend genannten Verbindungen der allgemeinen Formel XII in sehr verdünnten Mineralsäuren, wie Salzsäure, Bromwasserstoffsäure und/oder Schwefelsäure, oder Mischungen dieser wäßrigen Mineralsäuren mit Eisessig und/oder Ameisensäure bis zum Rückfluß,
- Abkühlen des Reaktionsansatzes,
- Waschen und Trocknen des Kristallisats
unter Erhalt der Verbindung der allgemeinen Formel Ib oder ihrer Tautomeren, worin Alk* n-Butylen, X Mercapto und A und R₅ dasselbe wie oben bedeuten.

6. Verfahren zur Herstellung der Verbindungen der allgemeinen Formel lb nach Anspruch 1, worin Alk* Alkylen C₄-C₁₂ unverzweigt und verzweigt, mit Ausnahme von 3-Methylpropylen (-CH₂-CH₂-CH(CH₃)-)), X Mercapto und A wie in Anspruch 1 bezeichnet bedeuten, **gekennzeichnet durch**
- Umsetzung von 2-Alkoxycarbonylamino-1-carbonsäurealkylester der allgemeinen Formel XIII, 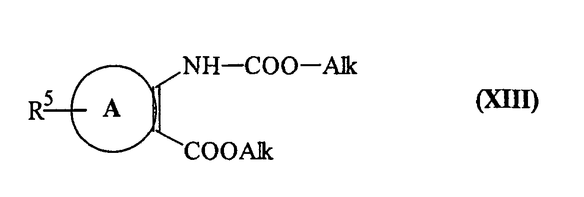
worin Alk Alkyl C₁-C₃ und A und R⁵ dasselbe wie oben bedeuten,
- mit Aminoalkanolen der allgemeinen Formel XIV,
**H**_{**2**}**NAlk*OH** **(XIV)**
worin Alk* Alkylen C₄-C₁₂ unverzweigt und verzweigt, mit Ausnahme von 3-Methylpropylen (-CH₂-CH₂-CH(CH₃)-)) bedeuten,
- Erwärmen
- Abkühlen des Reaktionsansatzes,
- Zugabe von Wasser und verdünnter Salzsäure bis etwa pH 4,
- Isolieren des Niederschlages, Waschen mit Wasser und Trocknen unter Erhalt der mehrcyclischen 3-(ω-Hydroxyalkyl)-pyrimidin-2,4(1H,3H)-dione der allgemeinen Formel XV, 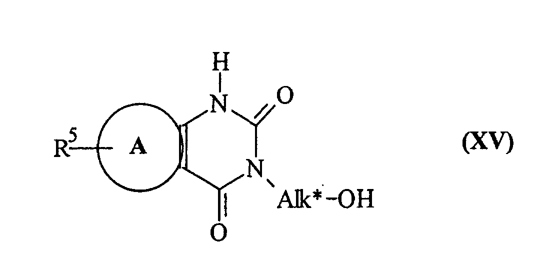 worin Alk* und A und R⁵ dasselbe wie oben bedeuten,
- Umsetzung der Verbindungen der allgemeinen Formel XV mit konzentrierter Salzsäure, Phosphortrichlorid oder Phosphoroxidchlorid oder mit konzentrierter Bromwasserstoffsäure, **durch** Erhitzen bis zum Rückfluß,
- Abkühlen des Reaktionsansatzes,
- Isolieren des Niederschlages, Waschen mit Wasser und Trocknen unter Erhalt der mehrcyclischen 3-(ω-Haloalkyl)-pyrimidin-2,4(1H,3H)-dione der allgemeinen Formel XVI, 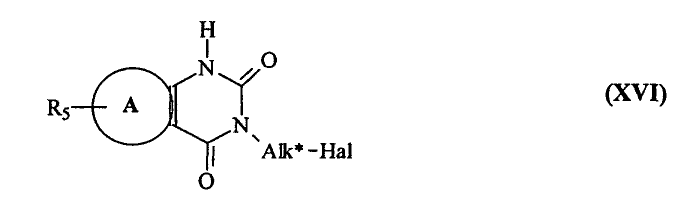 worin Hal Chlor oder Brom und Alk*, A und R⁵ dasselbe wie oben bedeuten,
- Umsetzung der Verbindungen der allgemeinen Formel XVI in einem hochsieden-, den, polaren Lösungsmittel mit Thioharnstoff
- Erhitzen unter Rückfluß,
- Abkühlen des Reaktionsansatzes,
- Zugeben von Wasser und verdünnter Natronlauge bis zum Erreichen eines alkalischen pH-Wertes,
- nach Aufklaren der Lösung filtrieren und
- Zugabe von verdünnter Salzsäure bis etwa pH 3,
- Isolieren des Niederschlages, Waschen mit Wasser und Trocknen unter Erhalt der Verbindungen der allgemeinen Formel Ib oder ihrer Tautomeren, worin Alk* Alkylen C₄-C₁₂ unverzweigt und verzweigt, mit Ausnahme von 3-Methylpropylen (-CH₂-CH₂-CH(CH₃)-)), X Mercapto und A und R⁵ dasselbe wie oben bedeuten.

7. Verfahren zur Herstellung der Verbindungen der allgemeinen Formel la bzw. Ib nach Anspruch 1, worin R³ bzw. X jeweils -SAlkCONHOH und R¹, R², Alk, R⁴, n, Alk*, A die Bedeutung wie in Anspruch 1 haben,
- durch Umsetzung von Verbindungen der allgemeinen Formel la bzw. Ib,
worin R³ und X Mercapto und R¹, R², R⁴, und n bzw. Alk*, A und R⁵ dasselbe wie oben bedeuten,
mit N-Hydroxyhalogencarbonsäureamiden,
- jeweils in Pyridin oder in acetonischer Lösung in Gegenwart einer Hilfsbase bei Raumtemperatur,
- Abdestillieren des Lösungsmittels,
- Zugabe von Wasser,
- Isolieren des Niederschlages, Waschen mit Wasser und Trocknen unter Erhalt der Verbindungen der allgemeinen Formel la bzw. Ib oder ihrer Tautomeren, worin R³ bzw. X -SALKCONHOH bedeutet.

8. Verfahren nach Anspruch 3, 4 oder 5, wobei die Mineralsäure Salzsäure, Bromwasserstoffsäure und/oder Schwefelsäure ist.

9. Verfahren nach Anspruch 4, wobei das aprotische, dipolare Lösungsmittel Dimethylformamid ist.

10. Verfahren nach Anspruch 3, wobei die Umsetzung der Verbindungen der allgemeinen Formel III mit Benzoylisothiocyanat als -C=S-Strukturelement übertragendem Agens in absolutiertem Aceton erfolgt.

11. Verfahren nach Anspruch 4, wobei die Umsetzung der Verbindungen der allgemeinen Formel VII mit den Verbindungen der allgemeinen Formel VIII in absolutiertem Dimethylformamid unter Zugabe von Kaliumcarbonat durch längeres Rühren bei Raumtemperatur erfolgt.

12. Verfahren nach Anspruch 3, **dadurch gekennzeichnet, daß** als das -C=S-Strukturelement übertragende Agens Thiophosgen, Thioharnstoff, Ammonium- oder Alkalithiocyanat/Salzsäure, 1,1'-Thiocarbonylbis-imidazol oder Benzoylisothiocyanat eingesetzt werden

13. Pharmazeutische Zubereitung, enthaltend einen oder mehrere Verbindungen nach Ansprüchen 1 und/oder 2 sowie übliche Hilfs- und Trägerstoffe

14. Pharmazeutische Zubereitungen zur Anwendung als Kollagenaseinhibitoren, **dadurch gekennzeichnet, dass** sie als Wirkstoff eine oder mehrere Verbindungen nach den Ansprüchen 1 und 2 enthalten.

## Claims

1. Polycyclic pyrimidine-2,4(1H,3H)-diones having functionalized alkyl radicals in 1-position and/or 3-position of general formulae Ia and Ib, 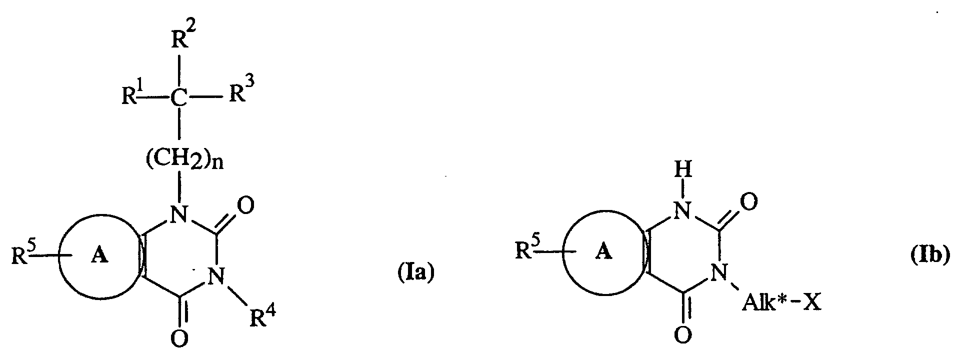 wherein
R¹ is hydrogen, methyl, ethyl,
R² is hydrogen or methyl,
R³ is mercapto or SAlkCONHOH,
Alk is branched or unbranched C₁-C₅ alkylene,
R⁴ is H, Alkyl, benzyl and phenyl,
n is 0, 1 or 2,
Alk* is branched or unbranched C₄-C₁₂ alkylene, with the exception of 3-methylpropylene,
X is mercapto or SAlkCONHOH,
A is anellated benzoring wherein
R⁵ is hydrogen, 6-methyl, 8-methyl, 6-fluorine, 6-chlorine, 6-bromine, 6-methylthio or 6,7-dimethoxy or thiophene ring annelated in the 2,3-position,
which is optionally substituted in the 4,5-position with dimethyl or condensed with a cyclopentene, cyclohexene or cycloheptene ring,
including the pharmaceutically suitable salts as well as the tautomers thereof.

2. Quinazoline-2,4(1H,3H)-diones having functionalized alkyl radicals in 1- and/or 3-position according to claim 1 of general formulae IIa and IIb, 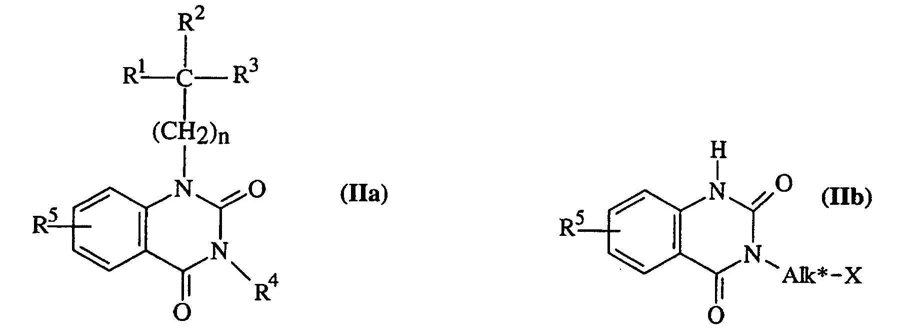 wherein R¹, R², R³, R⁴, Alk, Alk*, n and X are as defined in claim 1 including the pharmaceutically suitable salts as well as the tautomers thereof.

3. A method for the production of the compound of general formula Ia according to claim 1, wherein R⁴ = H, **characterized in that** the following steps are taken:
- reaction of 2-(alkenylamino)-1-carboxamides of general formula III 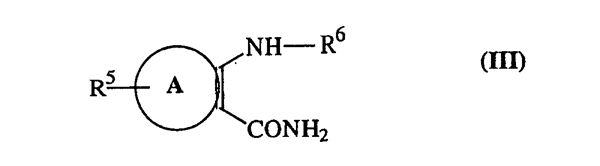 wherein R⁶ is C₃-C₆-alkenyl, and A is as defined in claim 1, with a C=S structural element-transferring agent in a polar, aprotic solvent,
- stirring the reaction batch,
- distilling off the solvent in a vacuum,
- addition of dilute alkali lye and moderate heating to about 60°C,
- separating undissolved components by filtration,
- cooling and acidifying the filtrate,
- heating the resulting compounds of general formula IV, 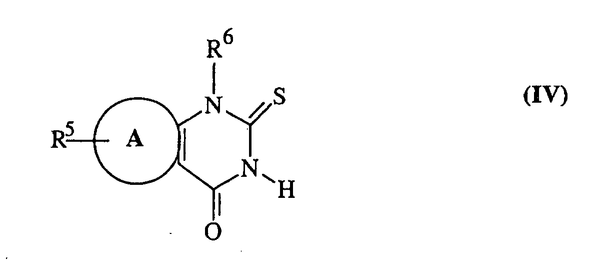 wherein A, R⁵ and R⁶ are as defined above,
- with concentrated mineral acid or mixtures thereof with glacial acetic acid and/or formic acid under reflux,
- cooling of the reaction batch,
- drying the resulting compound of general formula V, 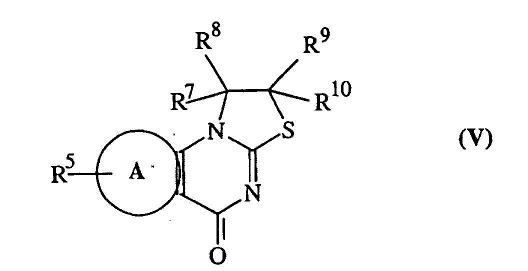 wherein
R⁷, R⁸, R⁹ and R¹⁰ are hydrogen, methyl or ethyl, and A and R⁵ are as defined above,
- or drying the resulting compound of general formula VI, 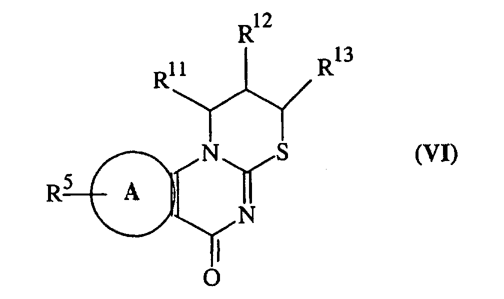 wherein R¹¹, R¹², R¹³ are hydrogen, methyl or ethyl and A and R⁵ are as defined above,
- in a vacuum exsiccator on potassium hydroxide or
- adding the above compounds by stirring to a dilute, aqueous sodium carbonate solution,
- isolating the compounds,
- heating these compounds in dilute mineral acid or mixtures thereof with glacial acetic acid and/or formic acid up to reflux,
- cooling the reaction batch,
- washing and drying the crystallizate thereby obtaining the compound of general formula Ia or their tautomers, wherein R⁴ is hydrogen without exception.

4. The method of producing the compounds of general formula Ia according to claim 1, wherein R³ is mercapto, and R⁴, n, A and R⁵ are as defined in claim 1,
by the following sequence of steps:
- reacting bicyclic and tricyclic 3-alkyl(and/or benzyl or phenyl)-pyrimidine-4(3H)-one-2(1H)-thiones of general formula VII, 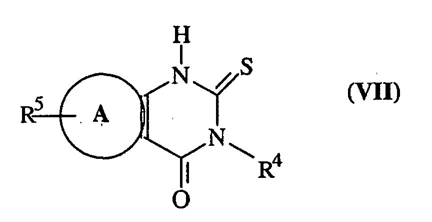
wherein R⁴ is H, Alk, benzyl, phenyl, and Alk, A and R⁵ are as defined above, with α,ω-dihalogen alkanes of general formula VIII,
Hal(CH₂)ₘHal (VIII)
wherein m is 2, 3 or 4, and Hal is chlorine, bromine or iodine,
- in an aprotic, dipolar solvent,
- addition of dilute hydrochloric acid,
- prolonged heating up to reflux,
- filtration of the hot reaction solution,
- cooling and storing the solution at 4°C,
- thereby obtaining the compounds of general formula Ia,
wherein R¹ = R² = H, R³ is mercapto, R⁴ is H, Alk, benzyl, phenyl, and Alk, A and R⁵ are as defined above,
or reaction of 2-ammoniumcarboxylic acid methyl ester thiocyanates of general formula IX, 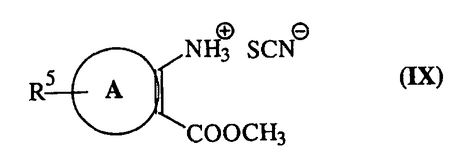 wherein A and R⁵ are as defined above,
with α,ω-dihalogen alkanes of general formula VIII,
wherein m is 2 or 3, and Hal is chlorine, bromine or iodine,
- heating the reactants with stirring up to reflux,
- cooling and isolating the precipitate,
- washing with diethyl ether and drying,
- dissolving the precipitate in water,
- filtrating the solution and adding dilute sodium hydroxide solution up to pH 10,
- isolating the precipitated precipitate and washing with water,
- drying the precipitate and intensive shaking using chloroform,
- isolating the precipitate and drying,
- recrystallization using an organic solvent,
thereby obtaining the compounds of general formula V, 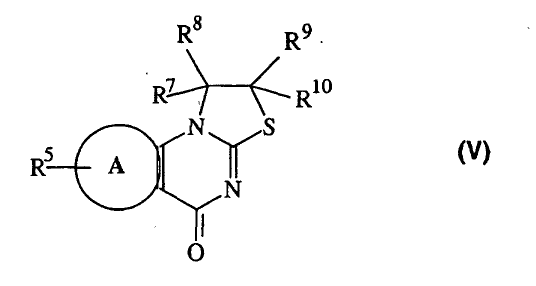 wherein R⁷, R⁸, R⁹ and R¹⁰ are hydrogen, and A and R⁵ are as defined above, obtained by reaction with dibromomethane, or the compound of general formula VI, 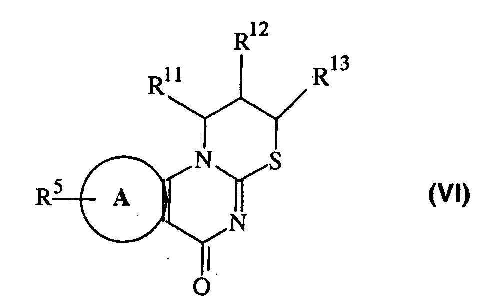 obtained by reaction with 1,3-dibromopropane, wherein R¹¹, R¹², R¹³ are hydrogen, and A and R⁵ are as defined above,
- heating the above-mentioned compounds of general formula V and/or VI in dilute mineral acid or mixtures thereof with glacial acetic acid and/or formic acid up to reflux,
- cooling the reaction batch,
- washing and drying the crystallisate thereby obtaining the compound of general formula Ia or the tautomers thereof,
wherein R¹, R², R⁴ are hydrogen, R³ is mercapto, n is 1 or 2, and A and R⁵ are as defined in claim 1.

5. The method for the production of the compounds of general formula Ib according to claim 1, wherein Alk is n-butylene, X is mercapto, and A is as defined in claim 1, **characterized by** reacting 2-isothiocyanato-1-carboxylic acid ester of general formula X, 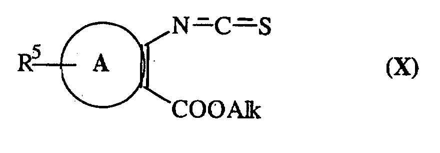 wherein Alk, A and R⁵ are as defined above,
- with 4-aminobutane-1-ol at room temperature and with intensive and prolonged stirring,
- adding water thereby obtaining the compounds of general formula XI, 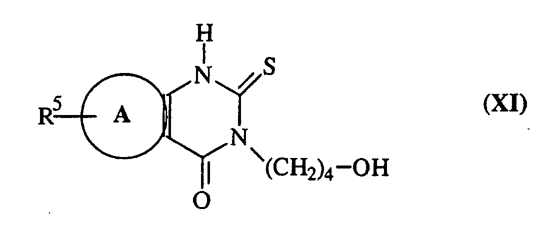 wherein A and R⁵ are as defined above,
- reacting the compounds of general formula XI with concentrated mineral acid or mixtures thereof with glacial acetic acid and/or formic acid up to reflux,
- cooling the reaction batch and isolating the crystallisate,
- adding aqueous sodium carbonate solution to the crystallisate up to about pH 9,
- isolating the crystallisate, washing with water and drying thereby obtaining the polycyclic 2,3,4,5-tetrahydro-7H-[1,3]thiazepino[2,3-a]pyrimidine-7-ones of general formula XII, 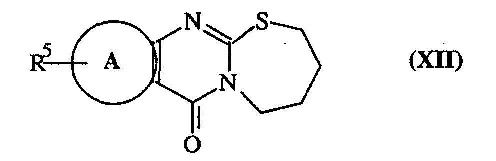 wherein A and R⁵ are as defined above,
- heating the above-mentioned compounds of general formula XII in highly dilute mineral acids, such as hydrochloric acid, hydrobromic acid and/or sulfuric acid or mixtures of these aqueous mineral acids with glacial acetic acid and/or formic acid up to reflux,
- cooling the reaction batch,
- washing and drying the crystallisate,
thereby obtaining the compound of general formula Ib or their tautomers, wherein Alk' is n-butylene, X is mercapto, and A and R⁵ are as defined above.

6. The method for the production of the compounds of general formula Ib according to claim 1, wherein Alk* is alkylene C₄-C₁₂ unbranched and branched, with the exception of 3-methyl-propylene (-CH₂-CH₂-CH(CH₃)-)), X is mercapto, and A is as defined in claim 1, **characterized by**
- reacting 2-alkoxycarbonylamino-1-carboxylic acid alkyl ester of general formula XIII, 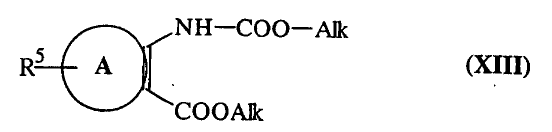 wherein Alk is alkyl C₁-C₃, and A and R⁵ are as defined above,
- with aminoalkanols of general formula XIV,
H₂NAlk*OH (XIV)
wherein Alk* is alkylene C₄-C₁₂ unbranched and branched, with the exception of 3-methylpropylene (-CH₂-CH₂-CH(CH₃)-)),
- heating,
- cooling the reaction batch,
- adding water and dilute hydrochloric acid up to about pH 4,
- isolating the precipitate, washing with water and drying thereby obtaining the polycyclic 3-(ω-hydroxyalkyl)-pyrimidine-2,4(1H,3H)-diones of general formula XV, 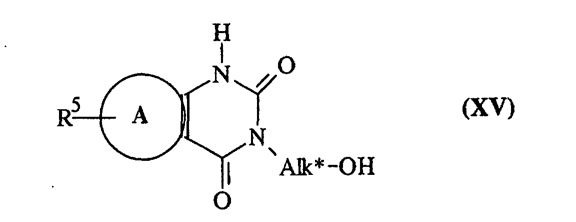 wherein Alk* and A and R⁵ are as defined above,
- reacting the compounds of general formula XV with concentrated hydrochloric acid, phosphorus trichloride or phosphorus oxychloride or with concentrated hydrobromic acid by heating up to reflux,
- cooling the reaction batch,
- isolating the precipitate, washing with water and drying thereby obtaining the polycyclic 3-(ω-haloalkyl)-pyrimidine-2,4(1H,3H)-diones of general formula XVI, 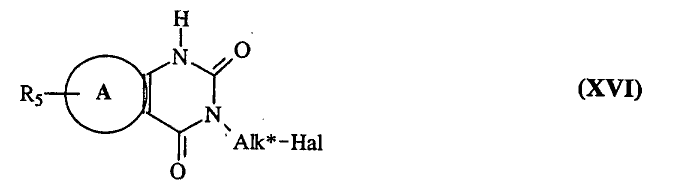 wherein Hal is chlorine or bromine and Alk*, A and R⁵ are as defined above,
- reacting the compounds of general formula XVI in a high-boiling, polar solvent with thiourea,
- heating under reflux,
- cooling the reaction batch,
- adding water and dilute caustic soda solution until an alkaline pH value has been reached,
- filtrating following clarification of the solution, and
- adding dilute hydrochloric acid up to pH 3,
- isolating the precipitate, washing with water and drying thereby obtaining the compounds of general formula Ib or the tautomers thereof, wherein Alk* is alkylene C₄-C₁₂ unbranched and branched, with the exception of 3-methylpropylene(-CH₂-CH₂-CH(CH₃)-)), X is mercapto, and A and R⁵ are as defined above.

7. The method for the production of the compounds of general formula Ia or Ib according to claim 1, wherein R³ and/or X are each -SAlkCONHOH, and R¹, R², Alk, R⁴, n, Alk*, A are as defined in claim 1,
- by reacting compounds of general formula Ia or Ib,
wherein R³ and X are mercapto, and R¹, R², R⁴ and n or Alk*, A and R⁵ are as defined above,
with N-hydroxyhalogencarboxamides,
- each in pyridine or in acetonic solution in the presence of an auxiliary base at room temperature,
- distilling off the solvent,
- adding water,
- isolating the precipitate, washing with water and drying thereby obtaining the compounds of general formula Ia or Ib or the tautomers thereof, wherein R³ or X are -SAlkCONHOH.

8. The method according to claim 3, 4 or 5, wherein the mineral acid is hydrochloric acid, hydrobromic acid and/or sulfuric acid.

9. The method according to claim 4, wherein the aprotic, dipolar solvent is dimethylformamide.

10. The method according to claim 3, wherein the compounds of general formula III are reacted with benzoyl isothiocyanate as -C=S structural element-transferring agent in absolute acetone.

11. The method according to claim 4, wherein the compounds of general formula VII are reacted with the compounds of general formula VIII in absolute dimethylformamide with the addition of potassium carbonate by prolonged stirring at room temperature.

12. The method according to claim 3, **characterized in that** the -C=S structural element-transferring agent used is thiophosgene, thiourea, ammonium or alkalithiocyanate/hydrochloric acid, 1,1'-thiocarbonylbis-imidazole or benzoyl isothiocyanate.

13. A pharmaceutical preparation, containing one or more compounds according to claims 1 and/or 2 as well as common auxiliary and carrier substances.

14. The pharmaceutical preparations for use as collagenase inhibitors, **characterized in that** they contain one or more compounds according to claims 1 and 2 as the active substance.

## Revendications

1. Pyrimidin-2,4(1H,3H)-diones polycycliques ayant des restes alkyle fonctionnalisés en position 1 et/ou 3 de formule générale Ia et Ib, 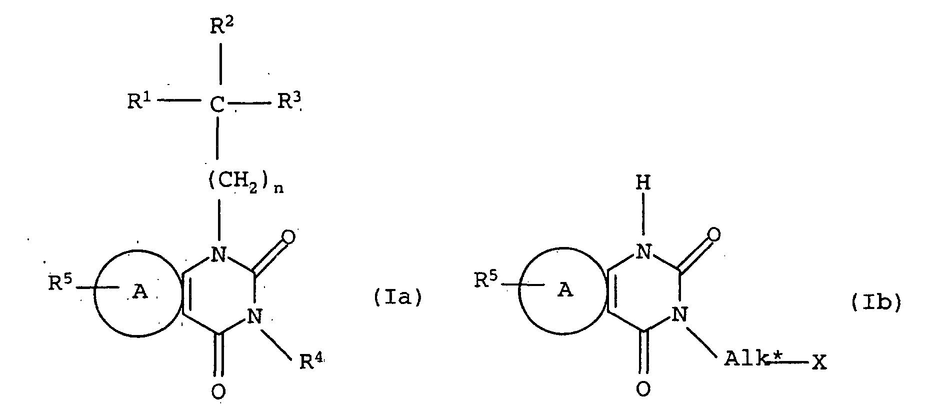 dans lesquelles :
R¹ représente l'hydrogène, un groupe méthyle, un groupe éthyle,
R² représente l'hydrogène ou un groupe méthyle,
R³ représente un groupe mercapto ou -SAlkCONHOH,
Alk représente un alkylène en C₁ à C₅ ramifié ou non ramifié,
R⁴ représente H, un groupe alkyle, benzyle ou phényle,
n est égal à 0, 1 ou 2,
Alk* représente un alkylène ramifié ou non ramifié en C₄ à C₁₂ à l'exception du 3-méthylpropylène,
X représente un groupe mercapto ou SAlkCONHOH,
A représente un cycle benzénique condensé,
R⁵ représentant l'hydrogène, un groupe 6-méthyle, 8-méthyle, 6-fluor, 6-chlore, 6-brome, 6-méthylthio ou 6,7-diméthoxy ou
un cycle thiophénique condensé en position 2,3,
substitué éventuellement en position 4,5 par un groupe diméthyle ou condensé avec un cycle cyclopenténique, cyclohexénique ou cyclohepténique,
ainsi que leurs sels si tautomères acceptables sur le plan pharmaceutique.

2. Quinazolin-2,4(1H,3H)-diones ayant des restes alkyle fonctionnalisés en position 1 et/ou 3 selon la revendication 1 de formule générale IIa et IIb, 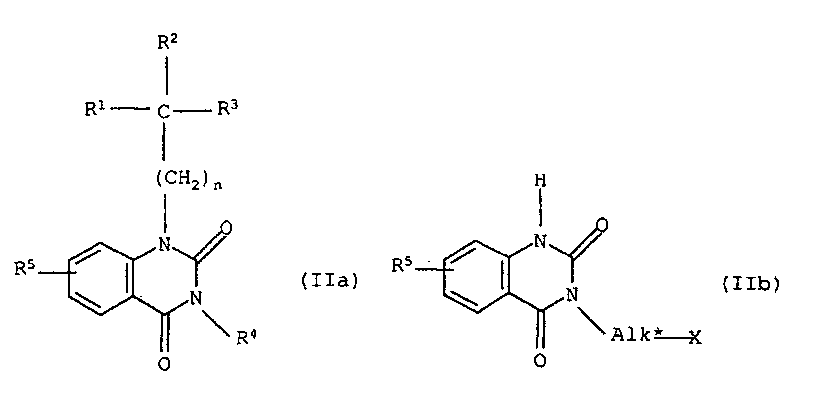 R¹, R², R³, R⁴, Alk, Alk*, n et X étant tels que dans la revendication 1, ainsi que leurs sels et tautomères acceptables sur le plan pharmaceutique.

3. Procédé de préparation du composé de formule générale Ia, selon la revendication 1, avec R⁴ = H, **caractérisé en ce que** l'on procède par les étapes suivantes :
- réaction des amides d'acide 2-(alcénylamino)-1-carboxyliques de formule générale III, 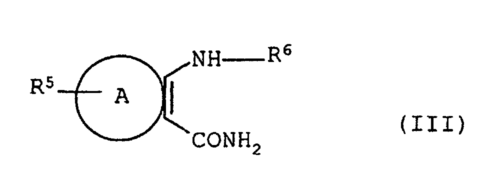
dans laquelle R⁶ représente un groupe alcényle en C₃ à C₆ et A est tel que défini dans la revendication 1, avec un agent transmettant l'élément structural C=S, dans un solvant aprotique polaire,
- agitation du mélange-réactionnel
- distillation du solvant sous vide,
- addition d'une lessive alcaline diluée et chauffage modéré jusqu'à environ 60°C,
- séparation des composants non dissous par filtration,
- refroidissement et acidification du filtrat,
- chauffage sous reflux des composés obtenus de formule générale IV, 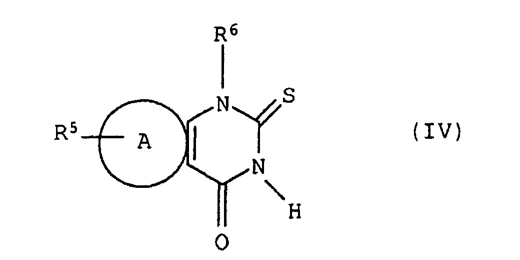 dans laquelle A, R⁵ et R⁶ ont les mêmes significations que précédemment,
- avec un acide minéral concentré ou des mélanges de celui-ci avec de l'acide acétique glacial et/ou de l'acide formique,
- refroidissement du mélange réactionnel,
- séchage du composé obtenu de formule générale V, 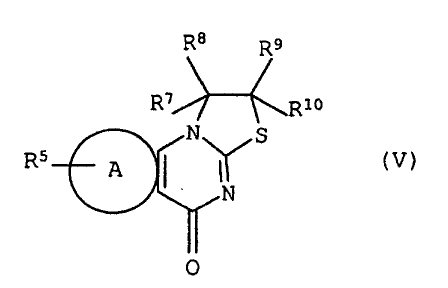
dans laquelle
R⁷, R⁸, R⁹ et R¹⁰ représentent l'hydrogène, un groupe méthyle ou éthyle et A et R⁵ ont les mêmes significations que précédemment,
ou le séchage du composé obtenu de formule générale VI, 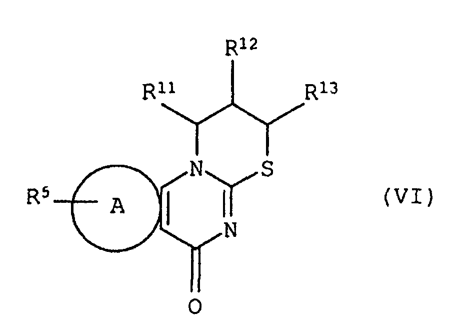 dans laquelle R¹¹, R¹², R¹³ représentent l'hydrogène, un groupe méthyle ou éthyle et A et R⁵ ont les mêmes significations que précédemment,
- dans un dessiccateur sous vide sur de l'hydroxyde de potassium ou
- introduction des composés susmentionnés dans une solution de carbonate de sodium aqueuse diluée,
- isolation des composés,
- chauffage jusqu'à reflux de ces composés dans un acide minéral dilué ou des mélanges de celui-ci avec de l'acide acétique glacial et/ou de l'acide formique,
- refroidissement du mélange réactionnel,
- lavage et séchage du cristallisat, ce qui permet d'obtenir le composé de formule générale Ia ou ses tautomères, où R⁴ représente exclusivement l'hydrogène.

4. Procédé de préparation des composés de formule générale Ia, selon la revendication 1, dans laquelle R³ représente un groupe mercapto et R⁴, n, A et R⁵ ont les mêmes significations que dans la revendication 1, **caractérisé par** la succession d'étapes de procédé suivantes :
- réaction de 3-alkyl(ou benzyl ou phényl)-pyrimidin-4(3H)-on-2(1H)thiones bi- ou tricycliques de formule générale VII, 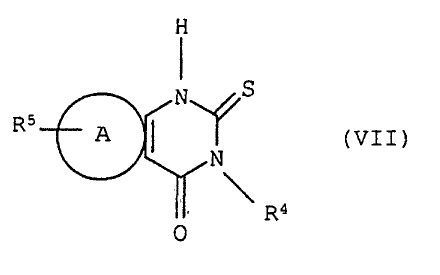
dans laquelle R⁴ représente H, un groupe Alk, benzyle, phényle et Alk, A et R⁵ ont les mêmes significations que précédemment,
avec des α,ω-dihalogénoalcanes de formule générale VII,
Hal(CH₂)ₘHal (VIII)
dans laquelle m est égal à 2, 3 ou 4 et Hal représente le chlore, le brome ou l'iode,
- dans un solvant aprotique dipolaire,
- addition d'acide chlorhydrique dilué,
- chauffage assez long jusqu'à reflux,
- filtration de la solution de réaction à chaud,
- refroidissement et maintien de la solution à 4°C,
- ce qui permet d'obtenir les composants de formule générale Ia,
dans laquelle R¹=R²=H, R³ représente un groupe mercapto, R⁴ représente un groupe H, Alk, benzyle, phényle et Alk, A et R⁵ ont les mêmes significations que précédemment,
ou
- réaction des thiocyanates d'ester méthylique d'acide ammonium-2-carboxylique de formule générale IX, 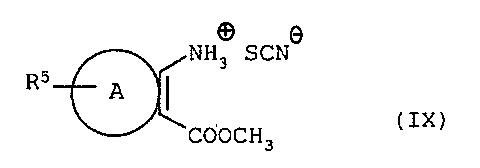
dans laquelle A et R⁵ ont les mêmes significations que précédemment,
avec des α,ω-dihalogénoalcanes de formule générale VIII,
dans laquelle m est égal à 2 ou 3 et Hal représente le chlore, le brome ou l'iode,
- chauffage jusqu'à reflux des partenaires réactionnels sous agitation,
- refroidissement et isolation du précipité,
- lavage avec de l'éther diéthylique et séchage,
- dissolution du précipité dans de l'eau,
- filtration de la solution et addition de lessive caustique diluée jusqu'à pH 10,
- isolation du précipité résultant et lavage avec de l'eau,
- séchage du précipité et agitation intensive avec du chloroforme,
- isolation du précipité et séchage,
- cristallisation avec un solvant organique,
- ce qui permet d'obtenir les composants de formule générale V, 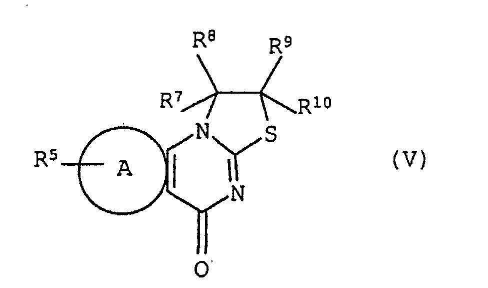
dans laquelle
R⁷, R⁸, R⁹ et R¹⁰ représentent l'hydrogène, et A et R⁵ ont les mêmes significations que précédemment, obtenus par réaction avec du dibromométhane,
ou le composé de formule générale VI, 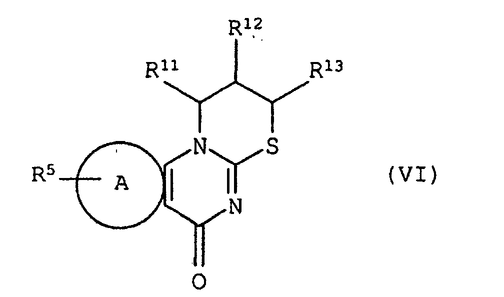 obtenu par réaction avec du 1,3-dibromopropane, dans laquelle R¹¹, R¹², R¹³ représentent l'hydrogène, et A et R⁵ ont les mêmes significations que précédemment,
- chauffage jusqu'à reflux des composés précédemment cités de formule générale V ou VI dans de l'acide minéral dilué ou des mélanges de celui-ci avec de l'acide acétique glacial et/ou de l'acide formique,
- refroidissement du mélange réactionnel,
- lavage et séchage du cristallisat, ce qui permet d'obtenir le composé de formule générale Ia ou ses tautomères,
dans laquelle R¹, R², R⁴ représentent l'hydrogène, R³ représente un groupe mercapto et n est égal à 1 ou 2 et A et R⁵ ont les mêmes significations que dans la revendication 1.

5. Procédé de préparation des composés de formule générale Ib, selon la revendication 1, dans laquelle Alk* représente un groupe n-butylène, X un groupe mercapto et A a la même signification que dans la revendication 1, **caractérisé par** la réaction de l'ester d'acide 2-isothiocyanato-1-carboxylique de formule générale X, 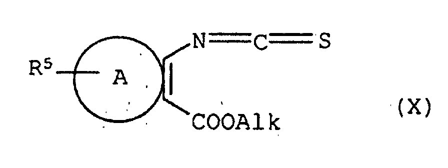 dans laquelle Alk, A et R⁵ ont les mêmes significations que précédemment,
- avec du 4-aminobutan-1-ol à température ambiante sous agitation intensive et assez longue,
- addition d'eau, ce qui permet d'obtenir les composés de formule générale XI, 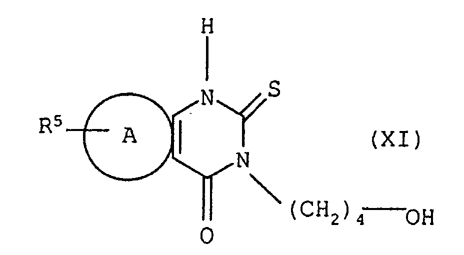
dans laquelle A et R⁵ ont les mêmes significations que précédemment,
- réaction des composés de formule générale XI dans de l'acide minéral concentré ou des mélanges de celui-ci avec de l'acide acétique glacial et/ou de l'acide formique jusqu'à reflux,
- refroidissement du mélange réactionnel et isolation du cristallisat,
- addition d'une solution de carbonate de sodium aqueuse au cristallisat jusqu'à un pH de 9 environ,
- isolation du cristallisat, lavage avec de l'eau et séchage, ce qui permet d'obtenir la 2,3,4,5-tétrahydro-7H-[1,3]thiazépino[2,3-a]pyrimidin-7-one de formule générale XII, 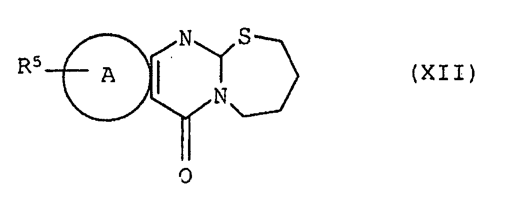
dans laquelle A et R⁵ ont les mêmes significations que précédemment,
- chauffage jusqu'à reflux des composés cités précédemment de formule générale XII dans des acides minéraux très dilués, comme l'acide chlorhydrique, l'acide bromhydrique et/ou l'acide sulfurique ou des mélanges de ces acides minéraux aqueux, avec de l'acide acétique glacial et/ou de l'acide formique,
- refroidissement du mélange réactionnel,
- lavage et séchage du cristallisat
ce qui permet d'obtenir le composé de formule générale Ib ou ses tautomères, dans laquelle Alk* représente un groupe n-butylène, X un groupe mercapto et A et R⁵ ont les mêmes significations que précédemment.

6. Procédé de préparation des composés de formule générale Ib, selon la revendication 1, dans laquelle Alk* représente un groupe alkylène en C₄ à C₁₂ non ramifié et ramifié, à l'exception du 3-méthylpropylène (-CH₂-CH₂-CH(CH₃)-)), X représente un groupe mercapto et A a la même signification que dans la revendication 1,
**caractérisé par**
- la réaction de l'ester alkyle d'acide 2-alcoxycarbonylamino-1-carboxylique de formule générale XIII, 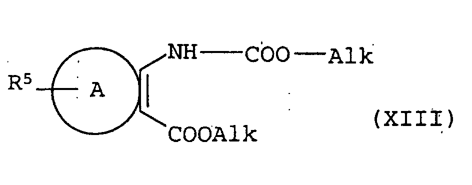 dans laquelle Alk représente un groupe alkyle en C₁ à C₃ et A et R⁵ ont les mêmes significations que précédemment,
- avec des aminoalcanols de formule générale XIV,
H₂NAlk*OH (XIV)
dans laquelle Alk* représente un groupe alkylène en C₄ à C₁₂ non ramifié et ramifié, à l'exception du 3-méthylpropylène (-CH₂-CH₂-CH(CH₃)-)),
- le chauffage,
- le refroidissement du mélange réactionnel,
- l'addition d'eau et d'acide chlorhydrique dilué jusqu'à un pH de 4,
- l'isolation du précipité, le lavage avec de l'eau et le séchage, ce qui permet d'obtenir les 3-(ω-hydroxyalkyl)-pyrimidin-2,4(1H,3H)-diones polycycliques de formule générale XV, 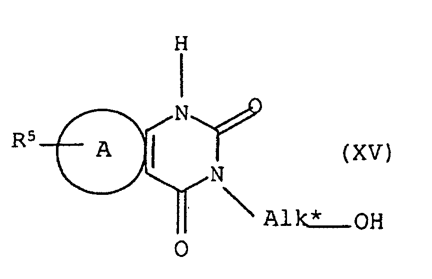 dans laquelle Alk* et A et R⁵ ont les mêmes significations que précédemment,
- la réaction des composés de formule générale XV avec de l'acide chlorhydrique concentré, du trichlorure de phosphore ou de l'oxychlorure de phosphore ou avec de l'acide bromhydrique concentré par chauffage jusqu'à reflux,
- le refroidissement du mélange réactionnel,
- l'isolation du précipité, le lavage avec de l'eau et le séchage, ce qui permet d'obtenir les 3-(ω-haloalkyl)-pyrimidin-2,4(1H,3H)-diones polycycliques de formule générale XVI, 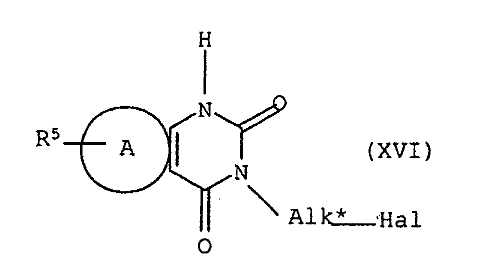 dans laquelle Hal représente le chlore ou le brome, Alk*, A et R⁵ ont les mêmes significations que précédemment,
- la réaction des composés de formule générale XVI dans un solvant polaire à point d'ébullition élevé avec de la thiourée,
- le chauffage sous reflux,
- le refroidissement du mélange réactionnel,
- l'addition d'eau et de lessive caustique diluée jusqu'à obtention d'un pH alcalin,
- après éclaircissement de la solution, la filtration et
- l'addition d'acide chlorhydrique dilué jusqu'à environ pH 3,
- l'isolation du précipité, le lavage avec de l'eau et le séchage, ce qui permet d'obtenir les composés de formule générale Ib ou leurs tautomères,
dans laquelle Alk* représente un groupe alkylène en C₄ à C₁₂ non ramifié et ramifié, à l'exception du 3-méthylpropylène (-CH₂-CH₂-CH(CH₃)-)), X représente un groupe mercapto et'A et R⁵ ont les mêmes significations que précédemment.

7. Procédé de préparation des composés de formule générale Ia ou Ib, selon la revendication 1, dans laquelle R³ ou X, respectivement, représente - SAlkCONHOH et R¹, R², Alk, R⁴, n, Alk* et A ont les mêmes significations que dans la revendication 1,
- par réaction de composés de formule générale Ia ou Ib, dans laquelle R³ et X représentent un groupe mercapto et R¹, R², R⁴ et n ou Alk*, A et R⁵ ont les mêmes significations que précédemment,
avec des amides d'acide N-hydroxyhalogénocarboxylique,
- respectivement dans de la pyridine ou dans une solution d'acétone en présence d'une base auxiliaire à température ambiante,
- distillation du solvant,
- addition d'eau,
- isolation du précipité, lavage avec de l'eau et séchage, ce qui permet d'obtenir les composés de formule générale Ia ou Ib ou leurs tautomères, dans laquelle R³ ou X représente un groupe -SAlkCONHOH.

8. Procédé selon la revendication 3, 4 ou 5, dans lequel l'acide minéral est l'acide chlorhydrique, l'acide bromhydrique et/ou l'acide sulfurique.

9. Procédé selon la revendication 4, dans lequel le solvant aprotique dipolaire est le diméthylformamide.

10. Procédé selon la revendication 3, dans lequel la réaction des composés de formule générale III s'effectue avec de l'isothiocyanate de benzoyle à titre d'agent transmettant l'élément structural -C=S- dans de l'acétone absolue.

11. Procédé selon la revendication 4, dans lequel la réaction des composés de formule générale VII avec les composés de formule générale VIII s'effectue dans du diméthylformamide absolu en ajoutant du carbonate de potassium sous agitation assez longue à température ambiante.

12. Procédé selon la revendication 3, **caractérisé en ce que** l'on met en oeuvre comme agent transmettant l'élément structural -C=S- le thiophosgène, la thiourée, le thiocyanate d'ammonium ou d'alcali/acide chlorhydrique, le 1,1'-thiocarbonylbis-imidazol ou l'isothiocyanate de benzoyl.

13. Composition pharmaceutique comprenant un ou plusieurs composés selon les revendications 1 et/ou 2 ainsi que des auxiliaires et supports usuels.

14. Compositions pharmaceutiques pour l'application en tant qu'inhibiteurs de la collagénase, **caractérisées en ce qu'**elles comprennent comme principe actif un ou plusieurs composés selon les revendications 1 et 2.
